(19) **Europäisches Patentamt / European Patent Office / Office européen des brevets**

(11) **EP 4 190 886 A1**

(12) **EUROPEAN PATENT APPLICATION**
published in accordance with Art. 153(4) EPC

(43) Date of publication:
**07.06.2023 Bulletin 2023/23**

(21) Application number: **21850755.6**

(22) Date of filing: **28.07.2021**

(51) International Patent Classification (IPC):
**C12M 1/00** (2006.01)     **C12M 1/34** (2006.01)
**G01N 35/00** (2006.01)     **C12Q 1/6851** (2018.01)
**C12Q 1/686** (2018.01)

(52) Cooperative Patent Classification (CPC):
**C12M 1/00; C12M 1/34; C12Q 1/6851; C12Q 1/686; G01N 35/00**

(86) International application number:
**PCT/JP2021/027866**

(87) International publication number:
**WO 2022/025104 (03.02.2022 Gazette 2022/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.07.2020 JP 2020130957**

(71) Applicant: **Ricoh Company, Ltd.**
**Tokyo 143-8555 (JP)**

(72) Inventors:
• **UNNO, Hirotaka**
 **Tokyo 143-8555 (JP)**
• **NAKAZAWA, Satoshi**
 **Tokyo 143-8555 (JP)**
• **YONEKAWA, Yuuki**
 **Tokyo 143-8555 (JP)**
• **JI, Yunong**
 **Tokyo 143-8555 (JP)**
• **OSAKI, Yusuke**
 **Tokyo 143-8555 (JP)**

(74) Representative: **Marks & Clerk LLP**
**15 Fetter Lane**
**London EC4A 1BW (GB)**

(54) **INFORMATION PROVISION DEVICE, INFORMATION PROVISION SYSTEM, INFORMATION PROVISION METHOD, AND PROGRAM**

(57)     An information provision device includes an acquisition unit and an information output unit. The acquisition unit acquires test result data indicating a Cq value of a well obtained by a test device performing a polymerase chain reaction (PCR) test on a standard device having the well in which a known number of copies of a nucleic acid are disposed. The information output unit outputs comparison information obtained by comparing information obtained from evaluation target data that is the evaluation target data of an evaluation target with information obtained from one or more pieces of the test result data different from the evaluation target data.

FIG. 4

## Description

Field of the Invention

The present invention relates to an information provision device, an information provision system, an information provision method, and a program. Priority is claimed on Japanese Patent Application No. 2020-130957, filed July 31, 2020, the content of which is incorporated herein by reference.

Description of Related Art

According to quantitative polymerase chain reaction (qPCR), the amount of nucleic acid in a specimen before amplification is estimated from a calibration curve and a Cq value of the specimen. A concentration range that can be estimated is usually a concentration range in which a calibration curve has linearity, and linearity over a wide concentration range is desired. qPCR performance includes not only linearity over a wide concentration range, but also amplification efficiency which is a slope of the calibration curve, a Cq value variation at each concentration, and repeatability. qPCR performance is influenced by differences in equipment, reagents, and primer design. There is a device for evaluating the performance of a test device (refer to Patent Document 1, for example).

It is desirable that the linearity of a calibration curve, a slope of the calibration curve, a Cq value variation, and the repeatability are the same in a case where the same specimen is measured under the conditions of the same device, the same reagent, and the same primer design. However, in reality, these are not always the same, and are often different. In particular, as can be seen from the fact that the linearity of the calibration curve, the slope of the calibration curve, and the Cq value variation are important, a Cq value is not used as a value for absolute comparison, but is handled as a value for relative comparison. The reason for this is that an accurate amount of nucleic acid for obtaining a calibration curve cannot be created, or a nucleic acid cannot be appropriately disposed. Temperature variations in a device, variations in a light detection side, and manufacturing variations in reagents are also major factors. As described above, the Cq value is handled as a relative value because the Cq value varies due to many factors. Therefore, even if the Cq value deviates before and after calibration of the same device, the Cq value is allowed without causing any particular problem.

As described above, the Cq value obtained as a measurement result of PCR varies. Therefore, PCR measurement results cannot be simply compared with past measurement results of the same device or measurement results of other test devices. Therefore, it may be difficult to determine, from a single measurement result, whether the test device from which the measurement result has been obtained is normal.

Summary of Invention

Technical Problem

An object of the present invention is to provide an information provision device, an information provision system, an information provision method, and a program capable of providing useful information for determining the normality of a test device that performs a PCR test.

Solution to Problem

According to an aspect of the present invention, there is provided an information provision device including an acquisition unit configured to acquire test result data indicating a Cq value of a well obtained by a test device performing a polymerase chain reaction (PCR) test on a standard device having the well in which a known number of copies of a nucleic acid are disposed; and an information output unit configured to output comparison information obtained by comparing information obtained from evaluation target data that is the test result data of an evaluation target with information obtained from one or more pieces of the test result data different from the evaluation target data.

Advantageous Effects of Invention

According to the present embodiment, it is possible to provide useful information for determining the normality of a test device that performs a PCR test.

Brief Description of the Drawings

FIG. 1 is a diagram showing the overall configuration of an information provision system according to an embodiment of the present invention.

FIG. 2 is a diagram showing an example of a standard plate according to the same embodiment.

FIG. 3 is a diagram showing an example of the number of copies of a template nucleic acid disposed in a well of the standard plate according to the same embodiment.

FIG. 4 is a block diagram showing the configuration of an information provision device according to the same embodiment.

FIG. 5 is a flowchart showing an operation of a test result data reception process of the information provision device according to the same embodiment.

FIG. 6 is a flowchart showing an operation of an analysis process of the information provision device according to the same embodiment.

FIG. 7 is a diagram showing a calculation method of an LOD and an LOQ according to the same embodiment.

FIG. 8 is a diagram showing a display example of analysis result information according to the same embodiment.

FIG. 9 is a diagram showing a display example of analysis result information according to the same embodiment.

FIG. 10 is a diagram showing an example of test result data and analysis information according to the same embodiment.

FIG. 11 is a diagram showing an example of test result data and analysis information according to the same embodiment.

FIG. 12 is a diagram showing an example of test result data and analysis information according to the same embodiment.

FIG. 13 is a diagram showing an example of test result data and analysis information according to the same embodiment.

FIG. 14 is a diagram showing an example of comparison of Cq value distributions according to the same embodiment.

FIG. 15 is a diagram showing an example of comparison of Cq value distributions according to the same embodiment.

FIG. 16 is a diagram showing a hardware configuration of the information provision device according to the same embodiment.

Detailed Description of the Invention

Hereinafter, embodiments of the present invention will be described in detail with reference to the drawings in some cases.

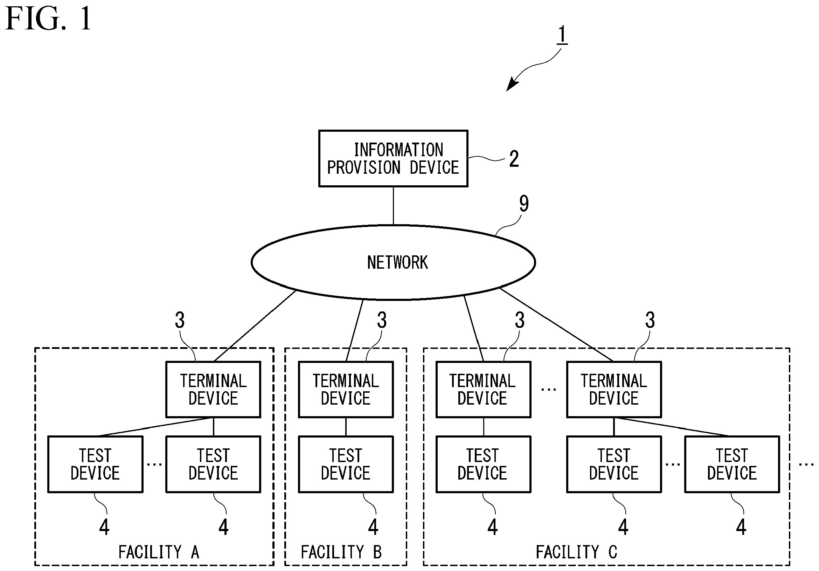

FIG. 1 is a diagram showing the overall configuration of an information provision system 1 according to an embodiment of the present invention. The information provision system 1 has an information provision device 2, a terminal device 3 and a test device 4. The information provision device 2 and the terminal device 3 are connected via a network 9. The network 9 is a public network such as the Internet. The network 9 may be a private network such as a leased line or a local area network (LAN), or a combination of a public network and a private network. The test device 4 is connected to the terminal device 3. The terminal device 3 and the test device 4 may be connected via the network 9. The terminal device 3 and the test device 4 may be integrated into one device. The test device 4 does not have to be connected to the terminal device 3. The information provision device 2 may be connected to the test device 4 directly or via the network 9. The information provision device 2 may be stand-alone. In this case, the information provision device 2 does not have to be connected to the terminal device 3 and the test device 4.

For example, a company that provides standard substances for a PCR test has the information provision device 2, and facilities A, B, C, ... that performs the PCR test has the terminal device 3 and the test device 4. The number of terminal devices 3 and test devices 4 included in each facility is arbitrary. Examples of facilities include, but are not limited to, companies, laboratories, and medical institutions. A facility may have the terminal device 3 or the test device 4 for each organization or a region of the facility. The facility that performs the PCR test may have the information provision system 1 or the information provision device 2.

The information provision device 2 acquires test result data, for example, by receiving, from the terminal device 3, the test result data obtained by the test device 4 performing a qPCR (real-time PCR) on a standard plate. The standard plate has a well in which a known number of copies of a nucleic acid are disposed. The information provision device 2 provides useful information for determining the normality of the test device 4 by outputting analysis result information obtained by performing analysis by using the acquired test result data. The analysis result information includes comparison information. Test result data for an evaluation target will be referred to as evaluation target data. The comparison information is obtained by comparing information obtained from the evaluation target data with information obtained from one or more pieces of test result data different from the evaluation target data. The comparison information includes information obtained from the evaluation target data and information obtained from the test result data compared with the evaluation target data. The comparison information includes information regarding whether or not the test device 4 from which the evaluation target data has been obtained is normal, and information regarding a well from which normal or abnormal Cq values have been obtained in the test device 4 from which the evaluation target data has been obtained. Hereinafter, the test result data to be compared with the evaluation target data will be referred to as comparison data.

The terminal device 3 is, for example, a computer device such as a personal computer, a tablet terminal, or a smart phone. The terminal device 3 communicates with the information provision device 2 via the network 9. The terminal device 3 acquires test result data obtained by the test device 4 performing a qPCR test on the standard plate and transmits the acquired test result data to the information provision device 2. The terminal device 3 receives the analysis result information output by the information provision device 2.

The test device 4 is a device that performs a real-time PCR test. Real-time PCR is a type of qPCR (quantitative PCR). In real-time PCR, amplification by PCR is measured over time (real time), and a template nucleic acid is quantified on the basis of an amplification rate obtained through the measurement. A template nucleic acid is a nucleic acid in which at least a base sequence in the primer region is determined. Examples of nucleic acids include, but are not limited to, deoxyribonucleic acid (DNA), and ribonucleic acid (RNA), complementary DNA (cDNA).

The standard plate is a device with a plurality of wells in which a specific number of copies of the template nucleic acid. The standard plate is an example of a standard device. The well of the standard plate may contain reagents such as primers, amplification reagents, and fluorescent probes in addition to template nucleic acids. The test result data received by the information provision device 2 indicates a Cq value measured for each well of the standard plate. The Cq value is synonymous with a Ct value (threshold cycle). The Cq value indicates the number of PCR cycles at which a constant amount of amplified product is obtained. A small Cq value indicates a large amount of template nucleic acid, and a large Cq value indicates a small amount of template nucleic acid.

The qPCR test includes the steps of nucleic acid extraction, addition of extract and amplification reagents, nucleic acid amplification, and data analysis. The information provision device 2 provides useful information for quality control of the test device 4 on the basis of results of the qPCR test performed by the test device 4 by using the standard plate. This ensures accuracy in the data analysis step. Normally, even if a specimen is measured under the same conditions, a Cq value varies depending on the test device 4, and thus it tends to be difficult to determine whether the test device 4 is normal from a single measurement result. The information provision device 2 of the present embodiment statistically analyzes information useful for determining whether the test device 4 is operating normally by comparing e qPCR test results using the standard plate, and provides analysis results to a facility. The facility can obtain comparison results by transmitting results of the qPCR test performed on the standard plate by the test device 4 to the information provision device 2. Consequently, it is easy for the facility to frequently check that a measurement system of the test device 4 is normal, and thus tests can always be performed by using a reliable measurement system. When performing a qPCR test, in a case where an operator adds a primer, an amplification reagent, and a fluorescent probe to the standard plate, the operator's skill can be measured by comparing test result data of different operators.

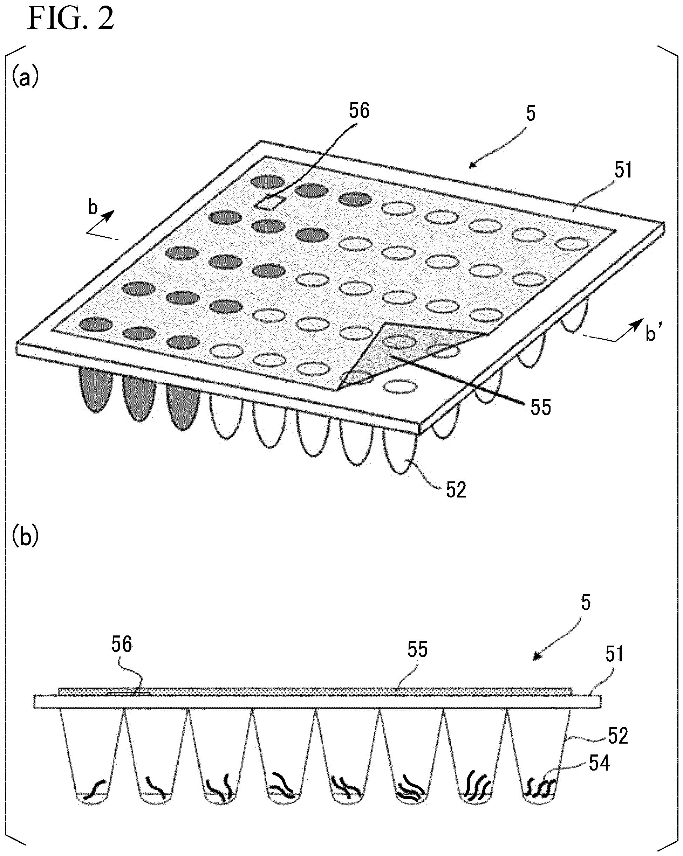

FIG. 2 is a diagram showing an example of a standard plate 5. FIG. 2(a) is a perspective view showing an example of the standard plate 5. FIG. 2(b) is a sectional view taken along line b-b' in FIG. 2(a). The standard plate 5 has a substrate 51 and a plurality of wells 52 formed in the substrate 51. The wells 52 of the standard plate 5 are filled with the same specific number of copies or a specific number of a plurality of kinds of copies of the template nucleic acid 54. The template nucleic acid 54 is a standard substance in a PCR test. The same type of template nucleic acid 54 is disposed in all the wells 52. The same type of the template nucleic acid has the same base sequence in the primer region. The template nucleic acid 54 may be a nucleic acid with a sequence which does not exist in nature or a nucleic acid with a sequence which exists in nature.

A small number of copies of the nucleic acid may be disposed in all or some of the wells 52 of the standard plate 5. The small number of copies is 200 copies or less where Poisson influence begins, or 50 copies or less where the influence becomes even greater. The number of copies of the template nucleic acid 54 smaller than the number of copies corresponding to a detection sensitivity required for the test device 4 may be disposed in all or some of the plurality of wells 52 of the standard plate 5. For example, it is assumed that the standard plate 5 has 96 wells 52 and the detection sensitivity required for the test device 4 is 20 copies. In this case, all 96 wells 52 may be provided with 10, 20, or 50 copies of template nucleic acids 54. Alternatively, 0, 5, 10, 20, 40, or 80 copies of template nucleic acids 54 may be disposed in each well group having 16 wells 52. Each well 52 may be further filled with one or more of a specific amount of primers, a specific amount of amplification reagents, and a specific amount of fluorescent probes. Hereinafter, disposing the same number of copies of the template nucleic acids 54 in all the wells 52 will also be referred to as entire-surface identical copy. The number of copies of the template nucleic acid 54 disposed in the well 52 will also be referred to as the number of copies of the well 52.

An opening part of well 52 is covered with a sealing member 55. Identification means 56 is disposed between the sealing member 55 and the substrate 51 and at a position other than the opening part of the well 52. The identification means 56 is, for example, a memory, an integrated circuit (IC) chip, or a radio frequency identification (RFID). The identification means 56 may be characters, barcodes, two-dimensional barcodes printed on a medium or the substrate 51. The identification means 56 stores plate identification information, plate type information, etc. of the standard plate 5. The plate identification information is information for identifying each standard plate 5. The plate type information is information indicating the type of standard plate 5. Depending on the type of standard plate 5, a combination of the type of template nucleic acid 54, the number of copies of a template nucleic acid 54 disposed in each well 52, the type and the amount of primer disposed in each well 52, and the type and the amount of the amplification reagent disposed in each well 52, and the type and the amount of fluorescent probe disposed in each well 52 can be specified. The number of copies includes a case of 0. The types and amounts of primers include a case where there is no primer. The types and amounts of amplification reagents include a case where there is no amplification reagent. The types and amounts of fluorescent probes include a case where there is no fluorescent probe. Plate type information can be specified from the plate identification information. Instead of or in addition to the plate type information, the identification means 56 may be store the type of template nucleic acid 54, the number of copies of a template nucleic acid 54 disposed in each well 52, the type of primer disposed in each well 52, the number of copies of a template nucleic acid 54 disposed in each well 52, the type and the amount of primer disposed in each well 52, and the type and the amount of the amplification reagent disposed in each well 52, and the type and the amount of fluorescent probe disposed in each well 52.

By using the standard plate 5, it is possible to check an in-plane distribution of the Cq value, the normality of the entire surface, and the detection sensitivity. The plane is the collection of all wells in a single plate. The accuracy of the test device 4 can be easily checked by performing measurement by using the standard plate 5 in which a small number of copies of the nucleic acid are disposed in some of the wells 52. Which type of standard plate 5 to use is selected according to the purpose.

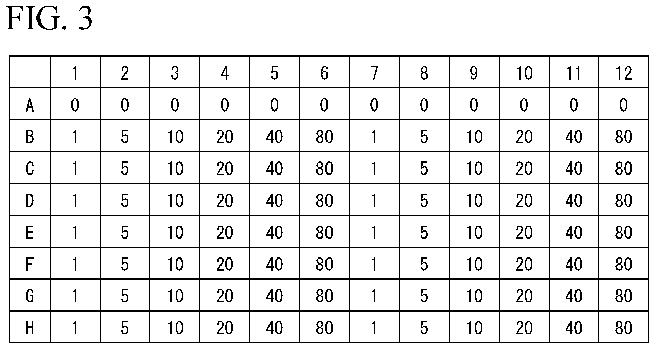

FIG. 3 is a diagram showing an example of the number of copies of the template nucleic acid 54 disposed in each well 52 of the standard plate 5. FIG. 3 shows a case where the standard plate 5 is a 96-well plate having 96 wells 52. Well identification information for identifying each well 52 is represented by a combination of row and column symbols. 'A' to 'H' in the leftmost column indicate row symbols for the 96-well plate, and ' 1' to ' 12' in the top row indicate column symbols for the 96-well plate. None of the wells 52 in the row A have no template nucleic acid. 1 copy of the template nucleic acid is disposed in the 1st and 7th columns of the rows B to H, 5 copies of the template nucleic acids are disposed in the 2nd and 8th columns, 10 copies of the template nucleic acids are disposed in the 3rd and 9th columns, 20 copies of the template nucleic acids are disposed 4th and 10th columns, 40 copies of the template nucleic acids are disposed in the 5th and 11th columns, and 80 copies of the template nucleic acids are disposed in the 6th and 12th columns.

Standard plate technology has allowed the production of the standard plates 5 in which an accurate amount of nucleic acids are disposed in each well 52. If the same Cq value can be obtained by testing the standard plate 5 by using the same test device 4, the same reagent, and the same primer, it is easy to find an abnormality in a case where there is a problem with any of the Cq values. For example, if one copy shows a certain Cq value and ten copies show another certain Cq value, it is possible to more accurately and reliably compare performances due to differences in the test device 4 or reagents. In other words, since an abnormality in the test device 4 or a reagent appears as a Cq value, it is possible to reliably manage the accuracy of the test device 4 or the reagent. In the present embodiment, the case where the standard device has a plate shape will be described, but the present invention is not limited to this. For example, the standard device may be a tube (for example, an octet tube) instead of the plate-shaped standard plate 5. The number of wells that the standard device has is not limited to 96, and may be, for example, 48 or 384.

Therefore, each facility refers to the information provided from the information provision device 2, and calibrates the test device 4 such that a Cq value for a predetermined number of copies is a fixed value when the same test device 4, the same model of the test device 4, or the different test device 4 regardless of model uses the same primer. Alternatively, each facility offsets a Cq value by referring to the information provided from the information provision device 2 in a case where the Cq value deviates before and after calibration as in conventional calibration. As described above, the facility can strictly manage the performance of the test device 4.

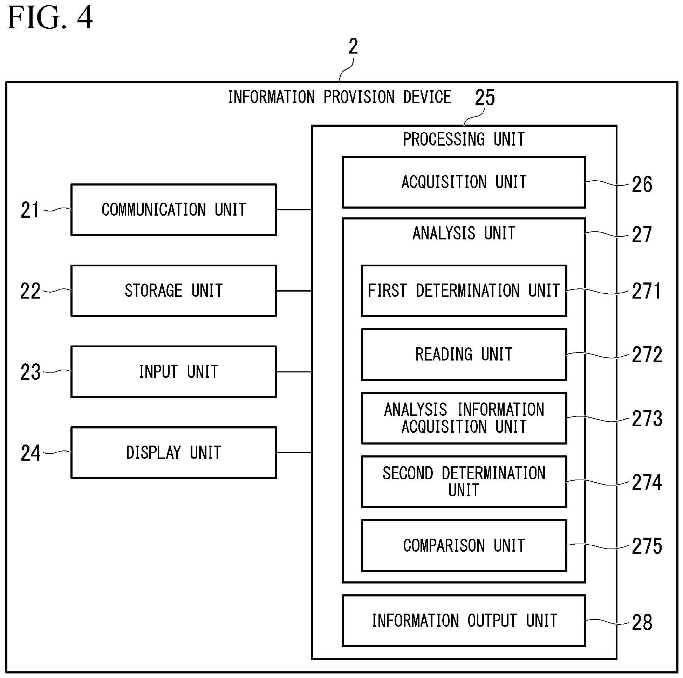

FIG. 4 is a block diagram showing a configuration of the information provision device 2. The information provision device 2 is implemented by, for example, one or more computer devices. The information provision device 2 may be a cloud server computer. The information provision device 2 has a communication unit 21, a storage unit 22, an input unit 23, a display unit 24, and a processing unit 25.

The communication unit 21 transmits and receives information to and from other devices via the network 9. The storage unit 22 stores various types of information including test result data and analysis result information. The storage unit 22 may further store standard plate information. The standard plate information corresponds to plate identification information or plate type information, and includes information regarding whether or not the standard plate 5 is an entire-surface identical copy plate, the type of template nucleic acid 54, the number of copies of a template nucleic acid disposed in each well 52, the type and the amount of primer disposed in each well 52, and the type and the amount of the amplification reagent disposed in each well 52, and the type and the amount of fluorescent probe disposed in each well 52. The information regarding the number of copies of template nucleic acids 54 disposed in each well 52 may be used as information regarding whether or not the standard plate 5 is an entire-surface identical copy plate. The input unit 23 is a keyboard, a mouse, buttons, a touch panel, or the like. The input unit 23 receives input of information by a user operation. The display unit 24 is a display that displays information.

The processing unit 25 has an acquisition unit 26, an analysis unit 27 and an information output unit 28. The acquisition unit 26 acquires test result data obtained by a PCR test performed by the test device 4 on the standard plate 5 and writes the acquired test result data into the storage unit 22. The acquisition unit 26 acquires test result data transmitted from the terminal device 3 connected via the network 9. The information provision device 2 may acquire test result data transmitted from the test device 4 connected via the network 9. In a case where the information provision device 2 is not connected to the terminal device 3 and the test device 4, the acquisition unit 26 may read the test result data from a computer-readable recording medium or the like, and acquire the test result data input by the input unit 23.

Attribute information is added to the test result data. The attribute information includes one or more pieces of information among the test date and time, test device identification information of the test device 4 that has performed the PCR test, a model of the test device 4 that has performed the PCR test, an owner of the test device 4 that has performed the PCR test, an installation location of the test device 4 that has performed the PCR test, an operator who has performed the PCR test, plate identification information of the standard plate 5 used for the PCR test, plate type information of the standard plate 5 used for the PCR test, and measurement conditions when the PCR test is performed. The attribute information includes, instead of or in addition to the plate type information, information regarding whether or not the standard plate 5 is an entire-surface identical copy plate, the type of template nucleic acid 54, the number of copies of the template nucleic acid 54 disposed in each well 52, the type and the amount of primer disposed in each well 52, and the type and the amount of the amplification reagent disposed in each well 52, and the type and the amount of fluorescent probe disposed in each well 52. The attribute information is based on information read from the identification means 56 of the standard plate 5 by reading means (not shown) provided in the terminal device 3 or the test device 4, information generated in the test device 4, information stored in advance in the terminal device 3 or the test device 4, or information manually input to the terminal device 3 or the test device 4. The owner information indicates a facility, a combination of a facility and an organization, and the like.

The analysis unit 27 has a first determination unit 271, a reading unit 272, an analysis information acquisition unit 273, a second determination unit 274 and a comparison unit 275. The first determination unit 271 determines, for each piece of test result data acquired by the acquisition unit 26, whether each well 52 is normal or abnormal in the measurement in which the test result data has been obtained. The first determination unit 271 writes well normality information into the storage unit 22 in association with the test result data. The well normality information indicates whether each well 52 is normal or abnormal.

The reading unit 272 reads, from the storage unit 22, test result data to be used as evaluation target data and one or more pieces of test result data to be used as comparison data. The comparison data is test result data that is different from the evaluation target data in either or both of a timing at which the PCR test was performed and the test device 4 that has performed the PCR test.

The analysis information acquisition unit 273 performs a process of removing a Cq value of the well 52 indicated as being abnormal by the normality information from the information used, for analysis for each of the evaluation target data and the comparison data. The analysis information acquisition unit 273 acquires evaluation target data analysis information by analyzing the evaluation target data excluding the Cq value of the well 52 indicating abnormality, and acquires comparison data analysis information by analyzing the comparison data excluding the Cq value of the well 52 indicating abnormality. The evaluation target data analysis information and the comparative data analysis information are or more of, for example, a distribution of Cq values, an average of Cq values, a limit of detection (LOD), a limit of quantitation (LOQ), a slope of a calibration curve, and an intercept of a calibration curve. The distribution of Cq values is represented, for example, by a standard deviation, a relative standard deviation, or an interquartile range of Cq values. The distribution of the Cq values in the evaluation target data analysis information is an in-plane distribution represented as a standard deviation, a relative standard deviation, or an interquartile range of the Cq values in a case of using the standard plate 5 that is an entire-surface identical copy plate. The LOD is the minimum number of copies for which the template nucleic acid can be detected, and the LOQ is the minimum number of copies for which the Cq value can be measured. The evaluation target data analysis information includes well normality information of the evaluation target data.

The second determination unit 274 determines the normality of the terminal device 3 on the basis of one or more pieces of information indicated by the evaluation target data analysis information. The comparison unit 275 obtains comparison information by comparing information obtained from the evaluation target data analysis information and information obtained from the comparison data analysis information. In addition to the information used for the comparison, the comparison information may include information regarding whether or not the test device 4 from which the evaluation target data has been obtained is normal, and information regarding a well from which a normal or abnormal Cq value has been obtained in the test device 4 from which the evaluation target data has been obtained. Information used for comparison to obtain comparison information is one or more of a distribution of Cq values, an average of Cq values, an LOD, an LOQ, a slope of a calibration curve, and an intercept of a calibration curve. The normality of the test device 4 can be checked from various aspects by performing comparison by using these pieces of information. The normality can be checked with higher accuracy by performing comparison by using a plurality of pieces of information.

The information output unit 28 generates analysis result information in which the evaluation target data analysis information and the comparison data analysis information acquired by the analysis information acquisition unit 273, the determination result of normality from the second determination unit 274, and the comparison information acquired by the comparison unit 275, are set. The information output unit 28 outputs the generated analysis result information. The output includes transmission to other devices such as the terminal device 3, recording to the storage unit 22 or other recording media, display on the display unit 24, printing using a printing device (not shown), and the like. The information output unit 28 writes the analysis result information into the storage unit 22 in association with the test result data used as evaluation target data.

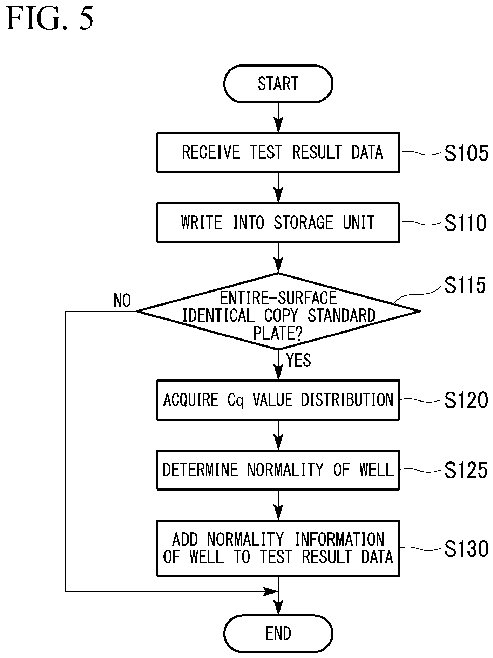

FIG. 5 is a flowchart showing an operation of a test result data reception process performed by the information provision device 2. The terminal device 3 transmits the test result data obtained by the PCR test performed by the test device 4 on the standard plate 5 to the information provision device 2. Attribute information is added to the test result data. The information provision device 2 performs a process shown in FIG. 5 on each piece of test result data.

The acquisition unit 26 of the information provision device 2 acquires test result data received by the communication unit 21 from the terminal device 3 (step S105). The acquisition unit 26 writes the received test result data into the storage unit 22 in association with reception time information (step S110). The reception time information indicates a time at which the information provision device 2 has received the test result data. On the basis of attribute information added to the test result data, the first determination unit 271 determines whether or not the standard plate 5 that is an entire-surface identical copy plate has been used (step 5115). In a case where the first determination unit 271 determines that the standard plate 5 that is an entire-surface identical copy plate has not been used (step S115: NO), the process is ended.

On the other hand, in a case where the first determination unit 271 determines that the standard plate 5 that is an entire-surface identical copy plate has been used (step S115: YES), an in-plane distribution of Cq values is obtained from the test result data (step S120). The first determination unit 271 compares the in-plane distribution of Cq values with a Cq value of each well 52 to determine the normality of each well 52 (step S125). In a case where the in-plane distribution of Cq values is a standard deviation $\sigma$ obtained from the Cq values of all the wells 52, the first determination unit 271 determines that the well 52 from which a Cq value with a standard deviation larger than a predetermined threshold value has been obtained is abnormal. Examples of the predetermined threshold value are, but are not limited to, $\sigma$, $2\sigma$, $3\sigma$, and the like. In a case where the in-plane distribution of the Cq values is within an interquartile range obtained from the Cq values of all the wells 52, the first determination unit 271 determines that the well 52 from which a Cq value deviating from the interquartile range by a predetermined amount or more has been obtained is abnormal. The first determination unit 271 determines that the wells 52 not determined as being abnormal are normal. The first determination unit 271 writes well normality information in association with the test result data written in the storage unit 22 in step S 110 (step S130). The well normality information indicates one or both of well identification information for the normal well 52 and well identification information for the abnormal well 52.

In a case where Cq values of the peripheral wells 52 are normal and Cq values of only some of the wells 52 are abnormal, the test device 4 is not abnormal, and it cannot be ruled out that the abnormality is caused by the influence of foreign matter or air bubbles. Therefore, in order not to use the wells 52 with abnormal Cq values for analysis, the well normality information is added to the test result data.

In a case where the standard plate 5 that is an entire-surface identical copy plate is not used, the first determination unit 271 may acquire information regarding the number of copies of each well 52 on the basis of the attribute information added to the test result data, and perform the processes in steps S120 and S125 for each well group of the same number of copies.

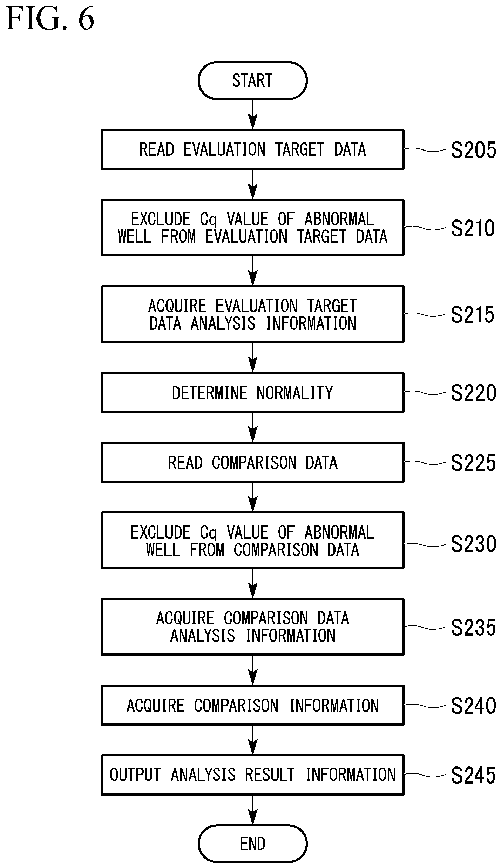

FIG. 6 is a flowchart showing an operation of an analysis process performed by the information provision device 2. The analysis process is performed, for example, in a case where an instruction is input by the input unit 23 after the process in FIG. 5 is performed, or at predetermined intervals.

The reading unit 272 reads one piece of the test result data stored in the storage unit 22 as evaluation target data (step S205). For example, the reading unit 272 reads new test result data written in the storage unit 22 in the process in FIG. 5 or test result data designated by the input unit 23 as evaluation target data. Alternatively, the reading unit 272 may read, as evaluation target data, test result data that matches evaluation target data selection conditions that are set in advance or input by the input unit 23, or may read test result data that has not been evaluation target data yet. In a case where there are a plurality of pieces of read test result data, the analysis unit 27 may set test result data designated by the input unit 23 from among the pieces of test result data as evaluation target data, or perform the processes in and after step S210 on each of the plurality of pieces of test result data. The reading unit 272 may receive evaluation target data selection conditions selected by the facility from the terminal device 3. Hereinafter, the test device 4 from which evaluation target data is obtained is described as an evaluation target test device.

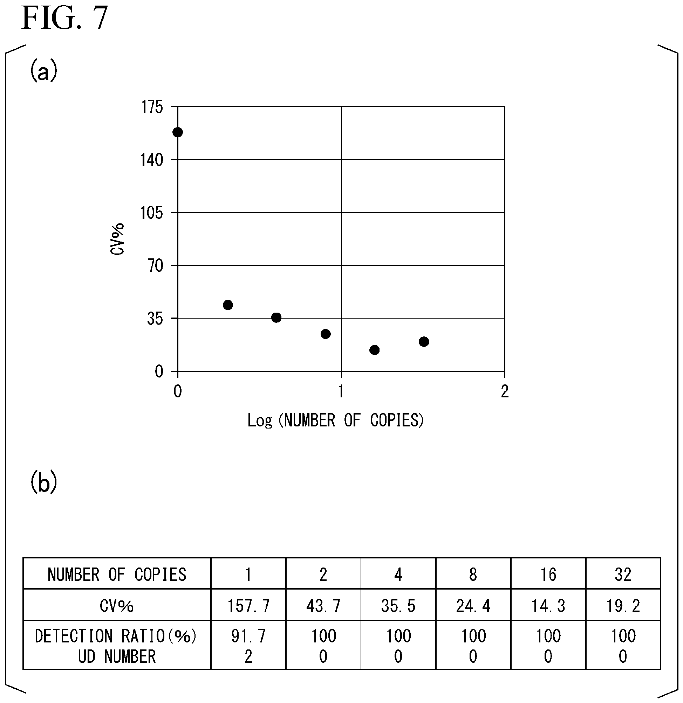

The analysis information acquisition unit 273 reads a Cq value of each well 52 from the evaluation target data, excluding the wells 52 indicated as being abnormal by the well normality information added to the evaluation target data (step S210). The analysis information acquisition unit 273 acquires evaluation target data analysis information on the basis of the information regarding the Cq value and number of copies of each well 52 (step S215). Specifically, the analysis information acquisition unit 273 reads information regarding the number of copies of each well 52 on the basis of the attribute information added to the evaluation target data. On the basis of the Cq value of each well 52 read in step S210 and the read information regarding the number of copies, the analysis information acquisition unit 273 calculates values of analysis items for one or more of an in-plane distribution of the Cq values, an average of the Cq values over the entire surface or for each number of copies, a distribution of the Cq values for each number of copies, an LOD, an LOQ, a slope of the calibration curve, and an intercept of the calibration curve, as evaluation target data analysis information. The in-plane distribution of the Cq values and the average of the Cq values over the entire surface are calculated in a case where the standard plate 5 that is an entire-surface identical copy plate has been used. The average of Cq values for each number of copies, the distribution of Cq values for each number of copies, the LOD, the LOQ, the slope of the calibration curve, and the intercept of the calibration curve are calculated in a case where the standard plate 5 that is not an entire-surface identical copy plate has been used. The standard plate 5 that is not an entire-surface identical copy plate is the standard plate 5 having wells 52 with a specific number of a plurality of kinds of copies. The in-plane distribution of Cq values or the distribution thereof for each number of copies are represented by a standard deviation, a relative standard deviation, and an interquartile range. The calibration curve is a straight line representing a relationship between the number of copies and a Cq value. An example of calculation of an LOD and an LOQ will be described later with reference to FIG. 7.

The analysis information acquisition unit 273 also adds the normality information of the evaluation target data and the Cq value of each well 52 read in step S210 to the evaluation target data analysis information. The analysis information acquisition unit 273 may add well abnormality information obtained from past test result data to the evaluation target data analysis information. In this case, the analysis information acquisition unit 273 reads the normality information from the past test result data in which the same test device identification information as the attribute information of the evaluation target data is set. The analysis information acquisition unit 273 specifies the well 52 determined as being abnormal at a predetermined frequency or more on the basis of the normality information set in the evaluation target data and the read past normality information. The analysis information acquisition unit 273 adds well abnormality information in which the well identification information of the specified well 52 is set to the evaluation target data analysis information.

The second determination unit 274 determines the normality of the terminal device 3 on the basis of the evaluation target data analysis information (step S220). For example, the second determination unit 274 determines whether the value of each analysis item in the evaluation target data analysis information is within the normal range. A normal range for each analysis item is stored in advance in the storage unit 22 according to, for example, the type and the number of copies of template nucleic acid 54, the type and the amount of reagent (a primer, an amplification reagent, and a fluorescent probe), and measurement conditions. For example, the second determination unit 274 may determine that the well 52 with 0 copies is normal in a case where a Cq value is not detected.

On the basis of the attribute information added to the test result data stored in the storage unit 22, the reading unit 272 reads one or more pieces of test result data that match the comparison data selection condition as comparison data (step S225). The comparison data selection conditions include at least a condition that the types and number of copies of the template nucleic acids indicated by the attribute information of the evaluation target data are the same. The comparison data selection conditions may include one or more pieces of information among the type and the amount of reagent (a primer, an amplification reagent, and a fluorescent probe), measurement conditions, test date and time, test device identification information, an owner, an installation location, a model, an operator, and a reception time. The type and the number of copies of template nucleic acid and the type and the amount of reagent may be indicated by plate identification information or plate type information. The comparison data selection conditions may be input by the input unit 23, or may be registered in advance in the storage unit 22 so as to correspond to some information included in the attribute information of the evaluation target data. Alternatively, the reading unit 272 may receive the comparison data selection condition selected in the facility from the terminal device 3.

By using test result data with different test dates and times in an evaluation target test device as the evaluation target data and the comparison data, it is possible to obtain comparison information from which whether performance has deteriorated due to changes over time can be determined. In this case, the comparison data selection conditions include a condition that the type and the number of copies of template nucleic acid, the type and the amount of reagent, measurement conditions, and test device identification information are the same as those for the evaluation target data, and the test date and time is earlier than the test date and time of evaluation target data.

By using test result data before and after relocation or before and after calibration of the evaluation target test device as the evaluation target data and the comparison data, it is possible to obtain, for example, comparison information from which whether performance has deteriorated after being relocated or calibrated can be determined. In this case, the test date and time of the evaluation target data is the date and time after relocation or calibration. The comparison data selection conditions include a condition that the type and the number of copies of template nucleic acid, the type and the amount of reagent, measurement conditions, and test device identification information are the same as those for the evaluation target data, and the test date and time is earlier than the test date and time of relocation or calibration.

In a case of obtaining comparison information for determining the performance compared with other test devices 4 of the same model as that the evaluation target test device, the comparison data selection conditions include a condition that are the type and the number of copies of template nucleic acid, and the type and the amount of reagent, measurement conditions, and a model of the test device 4 are the same as those for the evaluation target data, and the test device identification information is different from the evaluation target data. In a case of obtaining comparison information for determining the performance by comparing the evaluation target test device with the test devices 4 of other facilities, the comparison data selection conditions include a condition that the type and the number of copies of template nucleic acid, and the type and the amount of reagent, measurement conditions, and a model of the test device 4 are the same as those for the evaluation target data, and the facility is different from that for the evaluation target data.

In a case of obtaining comparison information for determining the skill of an operator, the comparison data selection conditions includes a condition that the type and the number of copies of template nucleic acid, and the type and the amount of reagent, measurement conditions, and a model of the test device 4 are the same as those for the evaluation target data, and the operator is different from that for the evaluation target data.

As described above, by appropriately setting the comparison data selection conditions, test result data in which evaluation target data and the test device 4, a model of the test device 4, a facility, a department (an organization in the facility), an operator, and an amplification reagent are the same or different can be used as comparison data. Test result data before and after relocation of the test device 4, test result data obtained by performing a PCR test within a predetermined period, and test result data obtained by performing a PCR test on a specific standard plate can be used as the evaluation target data and comparison data. Consequently, it is possible to select evaluation target data and comparison data depending on purposes such as whether the performance of the test device 4 has deteriorated due to the passage of time after calibration, whether the performance of the test device 4 has changed before and after relocation of the test device 4, how the performance of the test device 4 at one facility is compared with and different from that at another facility, and the skill of an operator, and perform comparison.

Subsequently, the analysis information acquisition unit 273 reads the Cq value of each well 52 except for the Cq value of the abnormal well 52 from each piece of the comparison data (step S230). The analysis information acquisition unit 273 acquires comparison data analysis information on the basis of the information of the Cq value of each well 52 of each comparison data read in step S230 and the number of copies of each well 52 of each comparison data (step S235). Specifically, the analysis information acquisition unit 273 reads information regarding the number of copies of each well 52 on the basis of the attribute information added to the comparison data. The analysis information acquisition unit 273 calculates one or more of the Cq value read in step S230, the distribution of the Cq values of all pieces of comparison data for each number of copies, each piece of comparison data for each number of copies and the average of the Cq values all pieces of comparison data, the LOD of each piece of comparison data, the LOQ of each piece of comparison data, the slope of the calibration curve of each piece of comparison data, and the intercept of the calibration curve of each comparison data as comparison data analysis information. The analysis information acquisition unit 273 may acquire these pieces of information on the basis of the analysis result information stored in the storage unit 22 to correspond to the comparison data. The analysis information acquisition unit 273 may cause information obtained from analysis result information corresponding to each piece of comparison data to be included in the comparison data analysis information.

The comparison unit 275 obtains comparison information by comparing the evaluation target data analysis information with the comparison data analysis information (step S240). A comparison item used for comparison is one or more pieces of information among a Cq value of a well, a distribution of Cq values, an average of Cq values, an LOD, an LOQ, a slope of a calibration curve, and an intercept of a calibration curve. The comparison unit 275 may select a comparison item depending on whether or not the standard plate 5 that is an entire-surface identical copy plate has been used for the evaluation target data.

For example, the comparison unit 275 calculates a deviation between a value of a comparison item obtained from the evaluation target data analysis information and a value of a comparison item obtained from the comparison data analysis information or an average of values of the comparison item. Alternatively, the comparison unit 275 calculates where a value of the item obtained from the evaluation target data analysis information is located in a distribution of values of the comparison item obtained from the comparison data analysis information. In a case where the comparison data selection conditions include the condition that the type and the amount of reagent and the measurement conditions are the same as those for the evaluation target data, the comparison unit 275 further determines whether the evaluation target test device is normal or abnormal on the basis of these calculation results.

It is assumed that a value of the comparison item is an average of Cq values for the number of copies N. N is preferably a number equal to or more than an LOQ. The comparison unit 275 calculates a deviation between the average of Cq values for the number of copies N in the evaluation target data and the average of Cq values for the number of copies N in all the pieces of comparison data. The comparison unit 275 determines that the evaluation target test device is normal in a case where the deviation is equal to or less than a predetermined threshold value, and determines that the evaluation target test device is abnormal in a case where the deviation is more than a threshold value. Alternatively, the comparison unit 275 determines that the evaluation target test device is normal in a case where an average of the Cq values for the number of copies N in the evaluation target data is included in the average of the Cq values $\pm$ the standard deviation $\sigma$ for the number of copies N in all the pieces of comparison data, and determines that the evaluation target test device is abnormal in a case where the average is not included. The comparison unit 275 may determine that the evaluation target test device is normal in a case where an average of the Cq values for the number of copies N in the evaluation target data is included in the interquartile range of the Cq values for the number of copies N in all the pieces of comparison data, and may determine that the evaluation target test device is abnormal in a case where the average is not included.

It is assumed that a value of the comparison item is Cq values of wells with the number of copies N. The comparison unit 275 determines that the evaluation target test device is normal in a case where all the Cq values of the respective wells with the number of copies N in the evaluation target data analysis information are included in the average of the Cq values $\pm$ the standard deviation $\sigma$ for the number of copies N in all the pieces of comparison data, and determines that the evaluation target test device is abnormal in a case where the Cq values are not included. Alternatively, the comparison unit 275 performs a significant difference test between a set of Cq values of the respective wells with the number of copies N in the evaluation target data and a set of Cq values of the respective wells with the number of copies N in all the pieces of comparison data. The comparison unit 275 determines that the evaluation target test device is normal in a case where there is no significant difference, and determines that the evaluation target test device is abnormal in a case where there is a significant difference.

It is assumed that a value of the comparison item is a standard deviation (distribution) of Cq values of wells with the number of copies N. The comparison unit 275 calculates a difference between the standard deviation of the Cq values of the wells with the number of copies N in the evaluation target data and the standard deviation of the Cq values of the wells with the number of copies N in all the pieces of comparison data. The comparison unit 275 determines that the evaluation target test device is normal in a case where the difference is within a predetermined threshold value, and determines that the evaluation target test device is abnormal in a case where the difference is more than the predetermined threshold value.

It is assumed that a value of the comparison item is an LOD. The comparison unit 275 calculates a deviation between the LOD in the evaluation target data and an average of LODs in all the pieces of comparison data. The comparison unit 275 determines that the evaluation target test device is normal in a case where the deviation is equal to or less than a predetermined threshold value, and determines that the evaluation target test device is abnormal in a case where the deviation is more than a threshold value. Alternatively, the comparison unit 275 determines that the evaluation target test device is normal in a case where the LOD in the evaluation target data is included in an average of the LODs $\pm$ the standard deviation $\sigma$ in all the pieces of comparison data, and determines that the evaluation target test device is abnormal in a case where the LOD is not included. The comparison unit 275 may determine that the evaluation target test device is normal in a case where the LOD in the evaluation target data is included in the interquartile range of the LOD in all the pieces of comparison data, and determine that the evaluation target test device is abnormal in a case where the LOD is not included. The comparison unit 275 similarly performs determination in a case where the comparison item is an LOQ, a slope of the calibration curve, or an intercept of the calibration curve.

In a case of using a plurality of comparison items, the comparison unit 275 may determine whether the evaluation target test device is normal or abnormal for each comparison item, and may determine a final determination that the evaluation target test device is abnormal in a case where it is determined that the evaluation target test device is abnormal for any or more that two predetermined number of the comparison items. Since the normality of the test device 4 can be checked from various aspects by using a plurality of comparison items, the normality of the test device 4 can be checked more accurately. The comparison unit 275 generates comparison information in which the values of the comparison item obtained from the evaluation target data analysis information, the values of the comparison item of the comparison data analysis information used for comparison, the average of the values, or the distribution of the values, and information regarding whether or not the evaluation target test device is normal, determined through the comparison, are set in the comparison information.

The information output unit 28 outputs analysis result information including the comparison information generated by the comparison unit 275, the evaluation target data analysis information and the comparison data analysis information generated by the analysis information acquisition unit 273, and the comparison data selection conditions (step S245). That is, the information output unit 28 writes the analysis result information into the storage unit 22 in association with the evaluation target data. In this case, the information output unit 28 may add information specifying the test result data used as the comparison data to the analysis result information and write the analysis result information to the storage unit 22. The information output unit 28 also generates analysis result notification information including the analysis result information, the evaluation target data, and the attribute information of the evaluation target data. The information output unit 28 may further set information obtained from the type of template nucleic acid 54, the number of copies in each well 52 of the standard plate 5 used for the measurement of the evaluation target data, the type and the amount of reagent, and the attribute information of the comparison data in the result notification information. The analysis result notification information may include display data for displaying the analysis result information in graphs or tables. For example, the display data can display analysis item values obtained from the evaluation target data analysis information and analysis item values and distributions in the comparison data analysis information used for comparison on the same graph or side by side in a table. The information output unit 28 transmits the analysis result notification information to the terminal device 3 of the facility specified by the attribute information of the evaluation target data. The terminal device 3 stores the received analysis result notification information and displays the analysis result notification information on a display.

In the facility, with reference to the analysis result notification information received by the terminal device 3, the evaluation target test device is calibrated such that a Cq value for a predetermined number of copies is a fixed value when the same test device 4, the same reagent, and the same primer are used. For example, information such as a difference in average of Cq values between an evaluation target test device indicated by the evaluation target data analysis information and the comparison data analysis information and another test device 4 is used for calibration of the evaluation target test device. Alternatively, in a case where a Cq value deviates before and after calibration as in conventional calibration, each facility refers to the provided information to offset the Cq value indicated by the test result of the evaluation target test device. Alternatively, the information output unit 28 of the information provision device 2 may output the analysis result information or the analysis result notification information by printing or displaying the information on the display unit 24, or may output the information to a terminal device of a company (not illustrated) that performs maintenance of the test device 4. An operator of the maintenance company checks the output analysis result information or analysis result notification information, and performs calibration and offset of the evaluation target test device at the facility. As described above, it is possible to strictly manage the performance of the test device 4. The facility performs processes such as cleaning for wells that are indicated to be abnormal by the well normality information. In a case where the comparison information indicates an abnormality, the facility calibrates a heat block, a lamp, and a detection system.

As described above, by using the comparison information generated by the information provision device 2, it is possible to more accurately compare test results and performance differences in certain measurement systems in different facilities, or test results and performance differences in certain measurement systems in the same facility, and thus more easily find defects in the test device 4 or reagents. By using a plurality of pieces of data acquired in the past by the same test device 4, it is possible to ascertain the current defect of the test device 4, and by using a plurality of pieces of data acquired at a plurality of facilities, it is also possible to determine a normal state of the test device 4 of each facility.

The terminal device 3 may add excluded well information to the test result data and transmit the data to the information provision device 2. The excluded well information indicates information regarding the well 52 that is not used for analysis. In this case, in step S210, the analysis information acquisition unit 273 reads the Cq value of each well 52 from the evaluation target data after further excluding the well 52 indicated by the excluded well information. The well 52 excluded from the evaluation target data may be a well of which abnormality is indicated by well normality information or well abnormality information added to the past test result data of the evaluation target test device. In this case, the test device 4 can use information specifying past test result data as the excluded well information.

The information provision device 2 may perform the processes in steps S225 to S240 for each comparison data group selected from each of a plurality of different comparison data selection conditions. After NO is determined in the process in step S115 in FIG. 5 or after the process in step S130, the information provision device 2 may perform the processes in steps S210 to S220 in FIG. 5 on the test result data, add the evaluation target data analysis information obtained through the process in step S215 and the determination result obtained through the process in step S220 to the test result data, and write the data into the storage unit 22.

FIG. 7 is a diagram for explaining a method of calculating an LOD and an LOQ. A method of detecting an LOQ is disclosed in, for example, the reference "A. Forootan, et al., "Methods to determine limit of detection and limit of quantification in quantitative real-time PCR (qPCR)," Biomolecular Detection and Quantification, Volume 12, June 2017, Pages 1 to 6". FIG. 7(a) is a graph showing a relationship between the number of copies and a CV value. FIG. 7(b) is a diagram showing a relationship between the number of copies, a CV value, a detection ratio, and a UD number. The number of wells for each number of copies is twenty-four for two measurements of the Le1 gene. UD indicates that no Cq value has been detected. According to the reference, the CV value shown in FIG. 7 is calculated by the following Equation (1). $SD_{Cq}$ is a standard deviation of the Cq values and E is the qPCR efficiency.

$$CV = \sqrt[2]{(1+E)^{(SD_{Cq})^2 \times \ln(1+E)} - 1} \qquad \cdots (1)$$

The analysis information acquisition unit 273 determines the minimum number of copies in which the number of non-detected wells 52 is equal to or less than a predetermined number as an LOD. The analysis information acquisition unit 273 also sets the minimum number of copies in which a CV value calculated by Equation (1) is equal to or less than a predetermined value as an LOQ. In the reference, the minimum number of copies in which the number of non-detected wells 52 is 0, that is a detection ratio is 100% is set as an LOD, the minimum number of copies in which a CV value is equal to or less than 35% is set as an LOQ. In the case of FIG. 7, the analysis information acquisition unit 273 determines the minimum number of copies "two" with a detection ratio of 100% as an LOD, and determines the minimum number of copies "eight" with a CV of 35% or less as an LOQ.

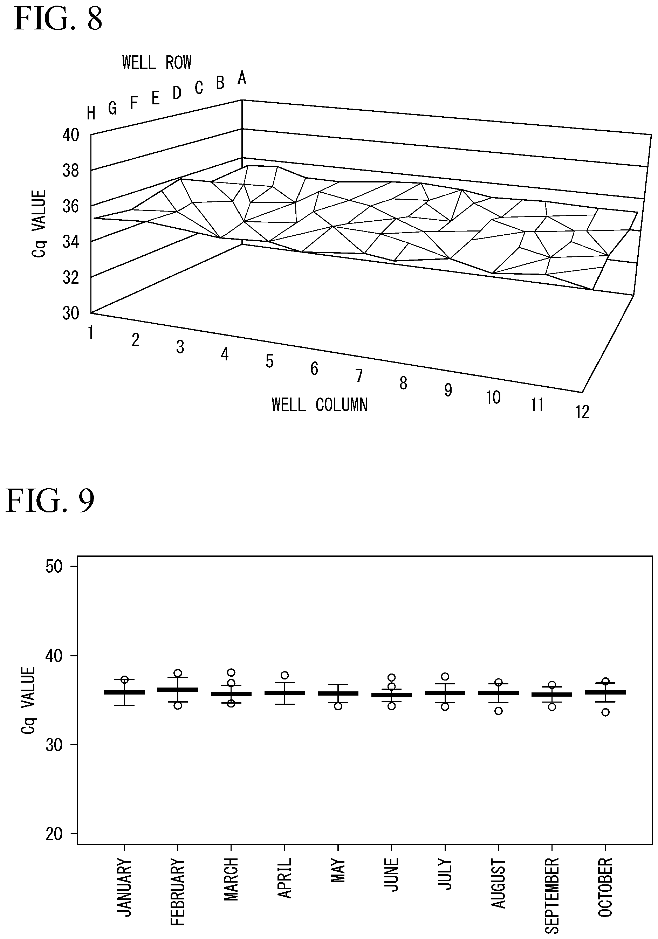

FIGS. 8 and 9 are diagrams showing display examples of analysis result information. The graph in FIG. 8 shows an in-plane distribution of Cq values indicated by the evaluation target data. The graph in FIG. 9 shows time-series changes in a distribution of Cq values of the evaluation target test device. The distribution of Cq values in October is obtained from the evaluation target data analysis information. A distribution of Cq values for each month from January to September is obtained from comparison data analysis information in which the test result data for each month of the evaluation target test device is used as comparison data.

Each facility uses the standard plate 5 in which a known number of copies of a template nucleic acid, a primer, an amplification reagent, and a fluorescent probe are set in each well 52, and transmits results of the test device 4 performing tests at predetermined intervals to the information provision device 2, and thus it is possible to check an in-plane distribution and receive feedback of information useful for monthly device accuracy control.

Next, application examples of the information provision system 1 will be described.

(Checking of detection sensitivity)

Here, an example of checking the detection sensitivity as the performance of the test device 4 will be described. The detection sensitivity is checked by a user such as a device manager at the facility at the time of periodic quality control of the test device 4, at the time of introduction of the test device 4, or before or after relocation of the test device 4. In a case of checking the detection sensitivity, it is desirable to check an in-plane distribution first. The in-plane distribution is checked in advance, and in a case where all wells are normal, it is guaranteed that all the wells will be used, and in a case where there is an abnormal well, the detection sensitivity can be obtained by excluding the abnormal well. Therefore, more accurate detection sensitivity information can be obtained.

In order to check that the in-plane distribution is normal, the standard plate 5 that is an entire-surface copy plate is used in which a specific small number of copies of a standard substance having a sequence which does not exist in nature are disposed in all wells 52. The number of copies disposed on the entire surface of the standard plate 5 is set according to purposes, such as two copies, which is the highest performance when a sequence which does not exist in nature is provided, ten copies, which is near the detection sensitivity, and fifty copies when a more reliable reaction is desired. The in-plane distribution, the normality of the entire surface, or the presence or absence of detection is checked by using the standard plate 5 having the number of copies according to the purpose.

For example, in a case where detection is required from twenty copies, it is desirable to check the in-plane distribution, the normality of the entire surface, and the presence or absence of detection obtained by the information provision device 2 analyzing test result data as evaluation target data, the test result data being obtained through a qPCR test performed by the test device 4 by using the standard plate 5 on which ten copies are disposed on the entire surface. In a case where it is desired to check only the normality of the entire surface and the presence or absence of detection, test result data obtained by measuring the standard plate 5 on which five copies or two copies of the template nucleic acids 54 are disposed may be used as evaluation target data.

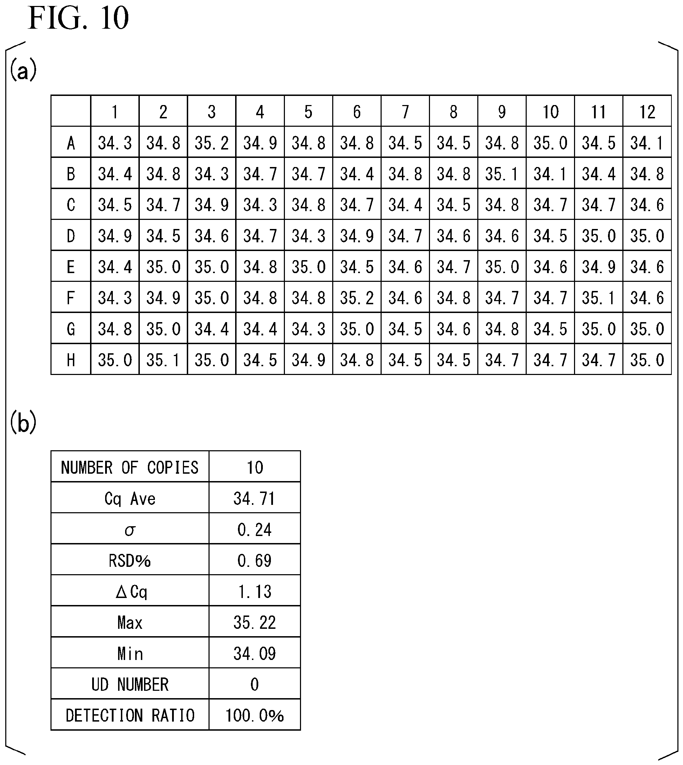

FIG. 10 is a diagram showing an example of test result data and obtained by the test device 4 performing a qPCR test by using the standard plate 5 with ten copies on the entire surface and analysis information thereof. Ten copies on the entire surface indicates that ten copies of the template nucleic acid 54 are disposed in all the wells 52. FIG. 10(a) shows test result data, and FIG. 10(b) shows analysis information obtained from the test result data shown in FIG. 10(a). The test result data show a Cq value for each well 52. The analysis information includes the number of copies of the template nucleic acid 54, the average of Cq values (Cq Ave), the standard deviation of the Cq values ($\sigma$), the relative standard deviation of the Cq values (RSD), the difference between the minimum Cq value and the maximum Cq value ($\Delta$Cq), the maximum Cq value (Max), the minimum Cq value (Min), a UD number, and a detection ratio. The UD number is the number of wells 52 in which no Cq value has been detected. The detection ratio is a percentage of wells 52 in which the Cq value has been detected.

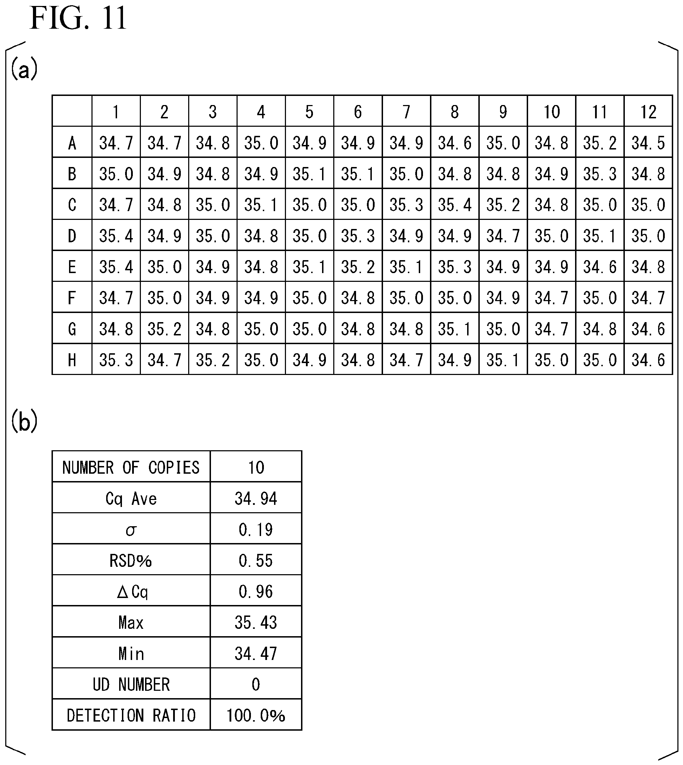

FIG. 11 is a diagram showing an example of test result data obtained by the same test device 4 performing the qPCR test under the same conditions as in FIG. 10 two months after the data in FIG. 10 has been obtained and analysis information thereof. FIG. 1 1(a) shows test result data, and FIG. 1 1(b) shows analysis information obtained from the test result data shown in FIG. 11(a).

Assume that FIG. 11(a) is evaluation target data, FIG. 11(b) is evaluation target data analysis information, FIG.

10(a) is comparison data, and FIG. 10(b) is comparison data analysis information. The second determination unit 274 determines that an average of the Cq values obtained from the evaluation target data analysis information in FIG. 1 1(b) is within a predetermined range (for example, 35 $\pm$ 0.5), a standard deviation of the Cq values is within a predetermined range (for example, $\sigma$ < 0.6), and a difference between the minimum Cq value and the maximum Cq value is within a predetermined range (for example, $\Delta$Cq < 2.0). The second determination unit 274 determines that all the wells 52 are normal because there is no well 52 indicating an abnormal Cq value and the UD number is 0. The comparison unit 275 compares the evaluation target data analysis information in FIG. 11(b) with the comparison data analysis information in FIG. 10(b), and determines that the evaluation target data analysis information has substantially the same accuracy as the comparison data analysis information obtained from the comparison data two months before.

As described above, the information provision device 2 can check whether or not the entire surface of the standard plate 5 is detected, use a distribution, an average, and a standard deviation of the Cq values as control values, compare the current distribution, average, and standard deviation of the Cq values with the previous distribution, average, and standard deviation of the Cq values, and check the normality of the current state of the test device 4. By using the standard plate 5 containing the template nucleic acid having a sequence that does not exist in nature, an effect of reducing the risk of contamination can be expected.

Next, the facility checks a state of detection sensitivity of the test device 4 by using the standard plate 5 on which the template nucleic acid having a sequence that does not exist in nature (artificial sequence) is disposed. The test device 4 performs a qPCR test by using the standard plate 5 shown in FIG. 3. Template nucleic acids with the number of copies of 0, 1, 5, 10, 20, 40, and 80 are disposed in 96 wells 52 of the standard plate 5.

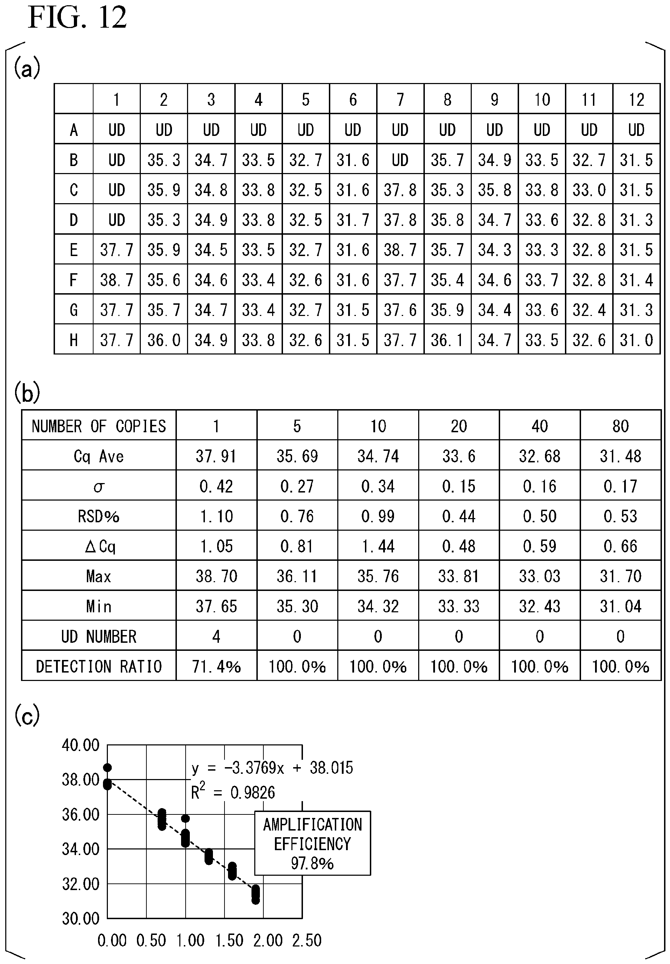

FIG. 12 is a diagram showing an example of test result data obtained by the test device 4 performing a qPCR test by using the standard plate 5 on which the template nucleic acid is disposed as shown in FIG. 3 after the data in FIG. 10 is obtained and analysis information thereof. FIG. 12(a) shows test result data, and FIGS. 12(b) and 12(c) show analysis information obtained from the test result data shown in FIG. 12(a). FIG. 12(b) shows information regarding the same analysis items as in FIGS. 10(b) and 11(b). FIG. 12(c) shows a calibration curve, a determination coefficient $R^2$, and amplification efficiency.

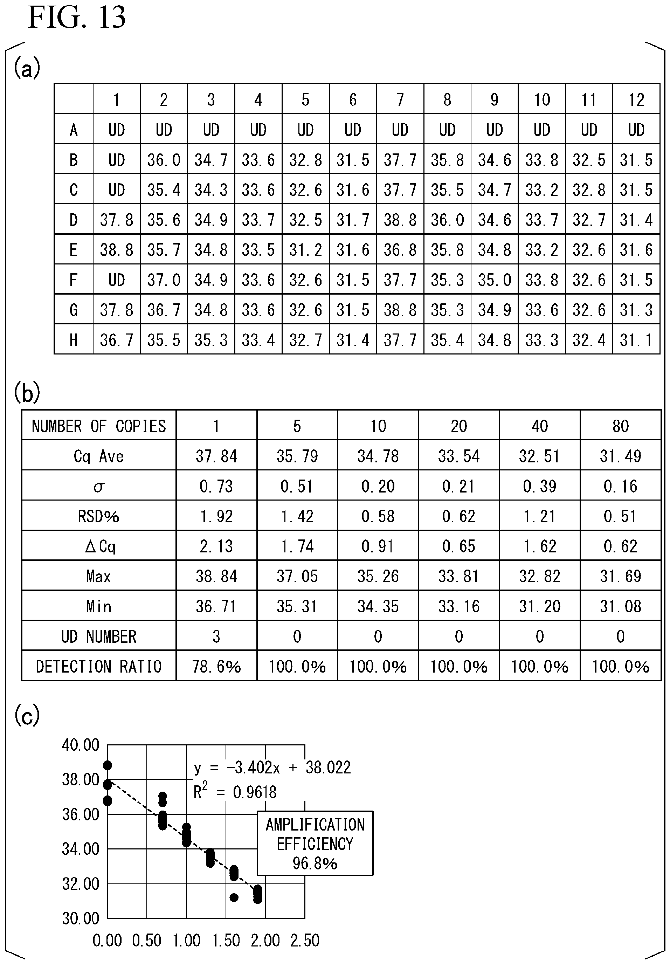

FIG. 13 is a diagram showing an example of test result data obtained by the same test device 4 performing a qPCR test under the same conditions as in FIG. 12 after the data in FIG. 11 is obtained and analysis information thereof. FIG. 13(a) shows test result data, and FIGS. 13(b) and 13(c) show analysis information obtained from the test result data shown in FIG. 13(a). FIG. 13(b) includes information regarding the same analysis items as in FIG. 12(b), and FIG. 13(c) shows the same analysis items as in FIG. 12(c).

Since it has been checked in advance that there is no problem with the Cq value distribution over the entire surface, the data of all the wells 52 can be used for evaluation. The second determination unit 274 of the information provision device 2 determines that the test is established because 0 copies are not amplified. The analysis information acquisition unit 273 determines that the detection sensitivity is from five copies because the detection ratio is 100% for five or more copies. The second determination unit 274 determines that a slope and an intercept (the intercept is a Cq value at one copy) of the calibration curve are within a predetermined range in addition to the detection sensitivity. The comparison unit 275 compares the slope and the intercept of the calibration curve with the slope and the intercept of the calibration curve in the comparison data that is the previous test result data, to determine whether or not the current state and performance of the test device 4 are normal. Consequently, the facility can perform normal tests and inspections by using the test device 4 that has been managed in a normal state. A template nucleic acid having a sequence that exists in nature may be used as a standard plate for checking states such as an in-plane distribution and the detection sensitivity, as long as comparison with previous data can be performed.

(Checking of state of test device 4)

Here, a state of the test device 4 is managed by checking whether or not the test device 4 can normally measure all 96 wells in qPCR/PCR. In this case, for example, the information provision device 2 receives test result data regarding results of the qPCR test performed by the evaluation target test device by using the standard plate 5 with ten copies on the entire surface at a predetermined frequency, and stores the received test result data in the storage unit 22. The information provision device 2 compares a distribution of Cq values in the latest test result data (evaluation target data) with a reference to manage whether or not the evaluation target test device is currently normal. As a reference for comparison, a distribution of the past Cq values obtained from the past test result data (comparison data) received from the evaluation target test device is used.

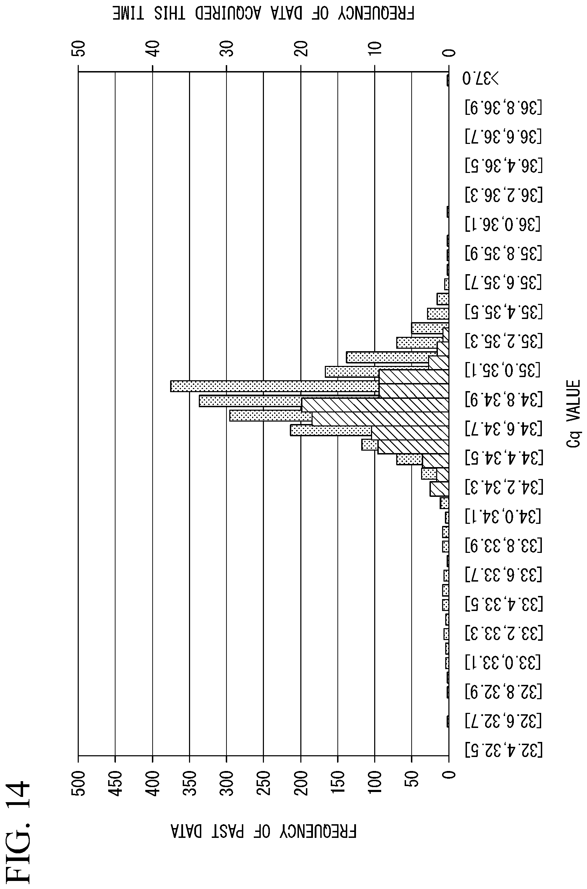

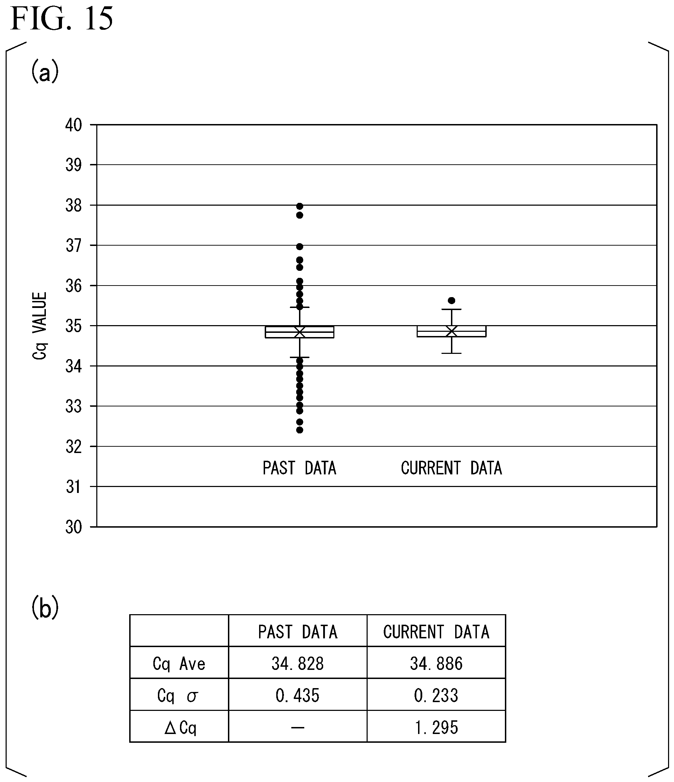

FIGS. 14 and 15 are diagrams showing comparative examples of distributions of Cq values measured in the past and Cq values measured this time. FIG. 14 shows a distribution with a histogram, and FIG. 15(a) shows a distribution with a boxplot. FIG. 15(b) shows an average (Cq Ave) and a standard deviation (Cq $\sigma$) of past and current Cq values, and a difference between the current maximum and minimum Cq values.

As shown in FIG. 14, the comparison unit 275 compares a distribution of Cq values obtained from the comparison data that is the past test result data of the evaluation target test device with a distribution of Cq values obtained from the evaluation target data that is the current test result data of the evaluation target test device. The comparison unit 275 determines that the state is normal if these distributions are more similar than a predetermined value, and determines that the state is abnormal if the distributions are not similar. The second determination unit 274 determines that the state is normal if an average of the current Cq values is within a predetermined range (for example, $35 \pm 0.5$) and a standard deviation of the Cq values is within a predetermined range (for example, $\sigma < 0.6$), and determines that the state is abnormal if otherwise. Alternatively, the comparison unit 275 determines that the state is normal in a case where a difference between the average of the past Cq values and the average of the current Cq values shown in FIG. 15 is within a predetermined range, a difference between the standard deviation of the past Cq values and the standard deviation of the current Cq values is within a predetermined range, and a difference between the current maximum and minimum Cq values is within a predetermined range. The predetermined range for determining normality may be a value determined according to internal standards of the facility or the like. In the above description, the comparison data is the past test result data of the evaluation target test device, but may be the past or current test result data of the test device 4 of another facility or the test device 4 of another model.

(Comparison of operator skills)

In a case of comparing operator skills, a standard plate 5 containing no primer, amplification reagent, or fluorescent probe is used. An operator prepares reagents such as a primer, an amplification reagent, and a fluorescent probe, and dispenses the reagents into each well 52 of the standard plate 5. The operator then performs a qPCR test on the standard plate 5 with the test device 4 according to the same PCR protocol. The terminal device 3 transmits test result data obtained by the qPCR test performed by the test device 4 to the information provision device 2. The information provision device 2 performs analysis by using test result data of different operators as evaluation target data and comparison data.

(Application to next-generation sequencers)

Next-generation sequencers (NGS) include amplicon NGS and the like in which a specific sequence is PCR-amplified with the test device 4 and the presence or absence or the amount of the amplified product is examined. In this case, after PCR amplification of the standard plate 5 with the test device 4, reagents for a test using NGS are prepared, the prepared reagents are added to the standard plate 5, and a test using NGS is performed. The information provision device 2 performs analysis by using test result data indicating test results using NGS instead of test result data of the test device 4.

(Systematization of quality control)

Analysis result information obtained by the information provision device 2, such as results of checking the detection sensitivity described above and results of checking a state of the test device 4, may be stored in the storage unit 22 or a server computer different from the information provision device 2 or a server computer on a cloud, and the analysis result information may be output in response to a request input by the input unit 23 or a request received from the terminal device 3 or the test device 4. Consequently, in each facility, at any time, it is possible to obtain results of comparing differences with other facilities, differences with other models, differences between the same models in the same facility, differences in the ability of operators, differences between amplification reagents, and sensitivities or states before and after relocation of the test device 4. An output destination of the analysis result output information may be a computer terminal (not shown) of a company or the like that manufactures, sells, or maintains the test device 4. Each facility or company that manufactures, sells, or maintains the test device 4 may freely select a factor desired to be compared, and in a case where there is no analysis result by the selected factor, the information provision device 2 may perform analysis and provide analysis results. As the information provision device 2 collects past test result data, accuracy improves and comparison based on more factors can be performed.

According to the information provision device 2 of the present embodiment, the test result data of the test device 4 of each facility can be collectively collected, and accuracy or quality of a state of the test device 4 can be analyzed, determined, and fed back to each facility. The information provision device 2 can collect test result data of the same model of the test devices 4 in different facilities, and provide information regarding a position at which the test result data obtained from an evaluation target test device among the test devices is distributed and to what extent characteristics are being obtained. A facility can check whether the current state of the test device 4 is normal compared with the past test result data and whether the test device 4 is operating normally through one qPCR measurement using a standard plate. The facility checks the normality and performance of the test device 4 on the basis of the information provided by the information provision device 2, and performs calibration if necessary. Therefore, it is possible to always perform tests by using a reliable measurement system. It is possible to store analysis results in a cloud or the like and provide the analysis results to companies that, for example, manufacture, sell, or maintain the test device 4 upon request.

The functions of the information provision device 2 and the test device 4 in the above-described embodiment may be realized by a computer. In that case, by recording a program for realizing the functions of the information provision device 2 and the test device 4 in a computer-readable recording medium, and causing a computer system to read and execute the program recorded on this recording medium, the functions may be realized. The "computer system" described herein includes hardware such as a central processing unit (CPU), an operation system (OS), and peripheral devices. The "computer-readable recording medium" is a portable medium such as a flexible disk, a magneto-optical disk, a read only memory (ROM), and CD-ROM, and a storage device such as a hard disk built into the computer system. The "computer-readable recording medium" may include a medium that dynamically stores a program for a short period of time, such as a communication line in a case of transmitting a program via a network such as the Internet or a communication line such as a telephone line, and a medium that stores the program for a certain period of time, such as a volatile memory inside a computer system that serves as a server or a client in that case. The program may be for realizing some of the above functions, or may be capable of realizing the above functions in combination with a program already recorded in the computer system. All or some of the functions of the information provision device 2 and the terminal device 3 are realized by using hardware such as an application specific integrated circuit (ASIC), a programmable logic device (PLD), or a field programmable gate array (FPGA).

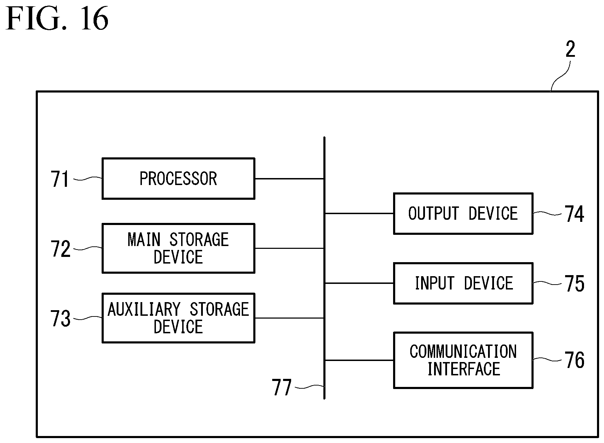

FIG. 16 is a block diagram showing an example of a hardware configuration of the information provision device 2. As shown in FIG. 16, the information provision device 2 has a processor 71, a main storage device 72, an auxiliary storage device 73, an output device 74, an input device 75 and a communication interface (communication I/F) 76. These units are connected to each other via a bus 77.

The processor 71 is a processing device that performs various types of control or calculations. For example, the processor 71 is a CPU. The processor 71 realizes various functions by executing an OS and programs stored in the main storage device 72 and the like. That is, the processor 71 functions as the processing unit 25 of the information provision device 2 by executing a performance evaluation program for a real-time PCR device. The processor 71 controls the overall operation of the information provision device 2. Here, the processor 71 is used as a device that controls the overall operation of the information provision device 2, but is not limited to this and may be, for example, an FPGA.

The performance evaluation program or various databases of the information provision device 2 do not necessarily have to be stored in the main storage device 72, the auxiliary storage device 73, or the like. The program and various databases of the information provision device 2 may be stored in another information processing device or the like connected to the information provision device 2 via a network such as the Internet, a local area network (LAN) or a wide area network (WAN). The information provision device 2 may acquire the program and the various databases from another information processing device and execute the program and the databases.

The main storage device 72 stores various programs and stores data and the like necessary for executing the various programs. The main storage device 72 has a ROM and a random access memory (RAM) (not shown). The ROM stores various programs such as a basic input/output system (BIOS). The RAM functions as a work area in which various programs stored in the ROM are loaded when the programs are executed by the processor 71. The RAM is not particularly limited and may be appropriately selected according to the purposes. Examples of the RAM include a dynamic random access memory (DRAM) and a static random access memory (SRAM).

The auxiliary storage device 73 is not particularly limited as long as various types of information can be stored, and may be appropriately selected according to the purposes. Examples thereof include a solid state drive and a hard disk drive. The auxiliary storage device 73 may be a portable storage device such as a compact disc (CD) drive, a digital versatile disc (DVD) drive, or a Blu-ray (registered trademark) disc (BD) drive.

A display, a speaker, or the like may be used as the output device 74. The display is not particularly limited, and any known display can be used as appropriate, such as a liquid crystal display and an organic EL display. The input device 75 is not particularly limited as long as various requests to the information provision device 2 can be received, and a known device may be used as appropriate, such as a keyboard, a mouse, and a touch panel. The communication interface 76 is not particularly limited, and a known interface may be used as appropriate, such as a wireless or wired communication device.

The processing functions of the information provision device 2 can be realized by the hardware configuration as described above.

The information provision device 2 may be implemented by a plurality of computer devices connected to a network. In this case, it is optional which of the plurality of computer devices implements each functional unit of the information provision device 2. The same functional unit may be realized by a plurality of computer devices. The test device 4 may have the functions of the information provision device 2.

The present invention includes the following aspects.

[1] An information provision device including an acquisition unit configured to acquire test result data indicating a Cq value of a well obtained by a test device performing a polymerase chain reaction (PCR) test on a standard device having the well in which a known number of copies of a nucleic acid are disposed; and an information output unit configured to output comparison information obtained by comparing information obtained from evaluation target data that is the test result data of an evaluation target with information obtained from one or more pieces of the test result data different from the evaluation target data.

[2] The information provision device according to [1], in which the same number of copies or the number of a plurality of kinds of copies of the nucleic acid are disposed in a plurality of the wells of the standard device.

[3] The information provision device according to [1] or [2], in which a small number of copies of the nucleic acid are disposed in at least some wells among a plurality of the wells of the standard device.

[4] The information provision device according to any one of [1] to [3], in which the number of copies of the nucleic acid smaller than the number of copies corresponding to a detection sensitivity required for the test device are disposed in at least some wells among a plurality of the wells of the standard device.

[5] The information provision device according to any one of [1] to [4], in which the comparison information includes one or both of information regarding whether or not the test device from which the evaluation target data has been obtained is normal, and information regarding the well from which a normal or abnormal Cq value has been obtained in the test device from which the evaluation target data has been obtained.

[6] The information provision device according to any one of [1] to [5], in which the information output unit further outputs one or both of information obtained from the evaluation target data and information obtained from one or more pieces of the test result data different from the evaluation target data.

[7] The information provision device according to any one of [1] to [6], in which the information obtained from the evaluation target data and the information obtained from one or more pieces of the test result data different from the evaluation target data, used for comparison by the information output unit, are one or more pieces of information among a distribution of Cq values, an average of the Cq values, the number of copies from which the nucleic acid is detectable, the number of copies from which the Cq value is measurable, a slope of a calibration curve, and an intercept of the calibration curve.

[8] The information provision device according to any one of [1] to [7], further including a determination unit configured to determine an abnormality of the well on the basis of the test result data for each piece of the test result data, in which the information output unit outputs the comparison information obtained by comparing the information obtained from the evaluation target data excluding the Cq value of the well determined as being abnormal with the information obtained from one or more pieces of the test result data different from the evaluation target data and excluding the Cq value of the well determined as being abnormal.

[9] The information provision device according to any one of [1] to [8], in which the information output unit provides the comparison information obtained by comparing the information obtained from the evaluation target data with the information obtained from one or more pieces of the test result data different from the evaluation target data in one or both of a time at which the PCR test has been performed and a test device that has performed the PCR test.

[10] The information provision device according to any one of [1] to [9], in which the evaluation target data is added with attribute information indicating one or more of identification of the test device, an owner of the test device, an installation location of the test device, a model of the test device, an operator who has performed the PCR test, a reagent used for the PCR test, the date and time at which the PCR test has been performed, identification of the standard device, and the type of the standard device, and the information output unit provides the comparison information obtained by comparing the information obtained from the evaluation target data with information obtained from one or more pieces of the test result data added with predetermined attribute information and different from the evaluation target data.

[11] The information provision device according to any one of [1] to [10], in which one or more of a predetermined amount of a primer, a predetermined amount of an amplification reagent, and a predetermined amount of a fluorescent probe are disposed in each well of the standard device.

[12] The information provision device according to any one of [1] to [11], in which the nucleic acid does not exist in nature.

[13] An information provision system including a reception unit configured to receive test result data indicating a Cq value of a well obtained by a test device performing a polymerase chain reaction (PCR) test on a standard device having the well in which a known number of copies of a nucleic acid are disposed; a storage unit configured to store the test result data received by the reception unit; and an information output unit configured to output comparison information obtained by comparing information obtained from evaluation target data that is the test result data of an evaluation target with information obtained from one or more pieces of the test result data different from the evaluation target data.

[14] An information provision method executed by an information provision device, including an acquisition step of acquiring test result data indicating a Cq value of a well obtained by performing a polymerase chain reaction (PCR) test on a standard device having the well in which a known number of copies of a nucleic acid are disposed; and

an information output step of outputting comparison information obtained by comparing information obtained from evaluation target data that is the test result data of an evaluation target with information obtained from one or more pieces of the test result data different from the evaluation target data.

[15] An information provision method executed by an information provision system, including a reception step of receiving test result data indicating a Cq value of a well obtained by a test device performing a polymerase chain reaction (PCR) test on a standard device having the well in which a known number of copies of a nucleic acid are disposed; a writing step of writing the test result data received in the reception step into a storage unit; and an information output step of outputting comparison information obtained by comparing information obtained from evaluation target data that is the test result data of an evaluation target with information obtained from one or more pieces of the test result data different from the evaluation target data.

[16] A program for causing a computer to function as the information provision device according to any one of [1] to [12].

[Reference Signs List]

1: Information provision system
2: Information provision device
3: Terminal device
4: Test device
5: Standard plate
9: Network
21: Communication unit
22: Storage unit
23: Input unit
24: Display unit
25: Processing unit
26: Acquisition unit
27: Analysis unit
28: Information output unit
51: Substrate
52: Well
54: Template nucleic acid
55: Sealing member
56: Identification means
71: Processor
72: Main storage device
73: Auxiliary storage device
74: Output device
75: Input device
76: Communication interface
77: Bus
271: First determination unit
272: Reading unit
273: Analysis information acquisition unit
274: Second determination unit
275: Comparison unit

Citation List

[Patent Document]

[Patent Document 1]
Japanese Unexamined Patent Application, First Publication No. 2020-054327

**Claims**

1. An information provision device comprising:

   an acquisition unit configured to acquire test result data indicating a Cq value of a well obtained by a test device performing a polymerase chain reaction (PCR) test on a standard device having the well in which a known number of copies of a nucleic acid are disposed; and
   an information output unit configured to output comparison information obtained by comparing information obtained from evaluation target data that is the test result data of an evaluation target with information obtained from one or more pieces of the test result data different from the evaluation target data.

2. The information provision device according to Claim 1, wherein
   the same number of copies or the number of a plurality of kinds of copies of the nucleic acid are disposed in a plurality of the wells of the standard device.

3. The information provision device according to Claim 1 or 2, wherein
   a small number of copies of the nucleic acid are disposed in at least some wells among a plurality of the wells of the standard device.

4. The information provision device according to any one of Claims 1 to 3, wherein
   the number of copies of the nucleic acid smaller than the number of copies corresponding to a detection sensitivity required for the test device are disposed in at least some wells among a plurality of the wells of the standard device.

5. The information provision device according to any one of Claims 1 to 4, wherein
   the comparison information includes one or both of information regarding whether or not the test device from which the evaluation target data has been obtained is normal, and information regarding the well from which a normal or abnormal Cq value has been obtained in the test device from which the evaluation target data has been obtained.

6. The information provision device according to any one of Claims 1 to 5, wherein
   the information output unit further outputs one or both of information obtained from the evaluation target data and information obtained from one or more pieces of the test result data different from the evaluation target data.

7. The information provision device according to any one of Claims 1 to 6, wherein
   the information obtained from the evaluation target data and the information obtained from one or more pieces of the test result data different from the evaluation target data, used for comparison by the information output unit, are one or more pieces of information among a distribution of Cq values, an average of the Cq values, the number of copies from which the nucleic acid is detectable, the number of copies from which the Cq value is measurable, a slope of a calibration curve, and an intercept of the calibration curve.

8. The information provision device according to any one of Claims 1 to 7, further comprising:

   a determination unit configured to determine an abnormality of the well on the basis of the test result data for each piece of the test result data, wherein
   the information output unit outputs the comparison information obtained by comparing the information obtained from the evaluation target data excluding the Cq value of the well determined as being abnormal with the information obtained from one or more pieces of the test result data different from the evaluation target data and excluding the Cq value of the well determined as being abnormal.

9. The information provision device according to any one of Claims 1 to 8, wherein
   the information output unit provides the comparison information obtained by comparing the information obtained from the evaluation target data with the information obtained from one or more pieces of the test result data different from the evaluation target data in one or both of a time at which the PCR test has been performed and a test device that has performed the PCR test.

10. The information provision device according to any one of Claims 1 to 9, wherein
    the evaluation target data is added with attribute information indicating one or more of identification of the test device, an owner of the test device, an installation location of the test device, a model of the test device, an operator who has performed the PCR test, a reagent used for the PCR test, date and time at which the PCR test has been performed, identification of the standard device, and the type of the standard device, and
the information output unit provides the comparison information obtained by comparing the information obtained from the evaluation target data with the information obtained from one or more pieces of the test result data added with predetermined attribute information and different from the evaluation target data.

11. The information provision device according to any one of Claims 1 to 10, wherein
one or more of a predetermined amount of a primer, a predetermined amount of an amplification reagent, and a predetermined amount of a fluorescent probe are disposed in each well of the standard device.

12. The information provision device according to any one of Claims 1 to 11, wherein
the nucleic acid does not exist in nature.

13. An information provision system comprising:

a reception unit configured to receive test result data indicating a Cq value of a well obtained by a test device performing a polymerase chain reaction (PCR) test on a standard device having the well in which a known number of copies of a nucleic acid are disposed;
a storage unit configured to store the test result data received by the reception unit; and
an information output unit configured to output comparison information obtained by comparing information obtained from evaluation target data that is the test result data of an evaluation target with information obtained from one or more pieces of the test result data different from the evaluation target data.

14. An information provision method executed by an information provision device, comprising:

an acquisition step of acquiring test result data indicating a Cq value of a well obtained by a test device performing a polymerase chain reaction (PCR) test on a standard device having the well in which a known number of copies of a nucleic acid are disposed; and
an information output step of outputting comparison information obtained by comparing information obtained from evaluation target data that is the test result data of an evaluation target with information obtained from one or more pieces of the test result data different from the evaluation target data.

15. An information provision method executed by an information provision system, comprising:

a reception step of receiving test result data indicating a Cq value of a well obtained by a test device performing a polymerase chain reaction (PCR) test on a standard device having the well in which a known number of copies of a nucleic acid are disposed;
a writing step of writing the test result data received in the reception step into a storage unit; and
an information output step of outputting comparison information obtained by comparing information obtained from evaluation target data that is the test result data of an evaluation target with information obtained from one or more pieces of the test result data different from the evaluation target data.

16. A program for causing a computer to function as the information provision device according to any one of Claims 1 to 12.

# FIG. 1

INFORMATION PROVISION DEVICE — 2

NETWORK — 9

TERMINAL DEVICE — 3

TEST DEVICE — 4

FACILITY A
FACILITY B
FACILITY C

EP 4 190 886 A1

# FIG. 2

(a)

(b)

FIG. 3

|   | 1 | 2 | 3 | 4 | 5 | 6 | 7 | 8 | 9 | 10 | 11 | 12 |
|---|---|---|---|---|---|---|---|---|---|----|----|----|
| A | 0 | 0 | 0 | 0 | 0 | 0 | 0 | 0 | 0 | 0 | 0 | 0 |
| B | 1 | 5 | 10 | 20 | 40 | 80 | 1 | 5 | 10 | 20 | 40 | 80 |
| C | 1 | 5 | 10 | 20 | 40 | 80 | 1 | 5 | 10 | 20 | 40 | 80 |
| D | 1 | 5 | 10 | 20 | 40 | 80 | 1 | 5 | 10 | 20 | 40 | 80 |
| E | 1 | 5 | 10 | 20 | 40 | 80 | 1 | 5 | 10 | 20 | 40 | 80 |
| F | 1 | 5 | 10 | 20 | 40 | 80 | 1 | 5 | 10 | 20 | 40 | 80 |
| G | 1 | 5 | 10 | 20 | 40 | 80 | 1 | 5 | 10 | 20 | 40 | 80 |
| H | 1 | 5 | 10 | 20 | 40 | 80 | 1 | 5 | 10 | 20 | 40 | 80 |

# FIG. 4

2

INFORMATION PROVISION DEVICE    25

PROCESSING UNIT

| | |
|---|---|
| ACQUISITION UNIT | 26 |

ANALYSIS UNIT — 27

| | |
|---|---|
| FIRST DETERMINATION UNIT | 271 |
| READING UNIT | 272 |
| ANALYSIS INFORMATION ACQUISITION UNIT | 273 |
| SECOND DETERMINATION UNIT | 274 |
| COMPARISON UNIT | 275 |

| | |
|---|---|
| INFORMATION OUTPUT UNIT | 28 |

| | |
|---|---|
| 21 | COMMUNICATION UNIT |
| 22 | STORAGE UNIT |
| 23 | INPUT UNIT |
| 24 | DISPLAY UNIT |

# FIG. 5

```
        ┌─────────────┐
        │    START    │
        └──────┬──────┘
               ↓
┌──────────────────────────────┐
│   RECEIVE  TEST  RESULT  DATA │────  S105
└──────────────┬───────────────┘
               ↓
┌──────────────────────────────┐
│   WRITE  INTO  STORAGE  UNIT  │────  S110
└──────────────┬───────────────┘
               ↓
           ╱ENTIRE-SURFACE╲
  NO      ╱IDENTICAL COPY STANDARD╲────  S115
◄────────◄        PLATE?          ╲
          ╲                       ╱
           ╲─────────┬───────────╱
                     ↓ YES
┌──────────────────────────────┐
│ ACQUIRE Cq VALUE DISTRIBUTION │────  S120
└──────────────┬───────────────┘
               ↓
┌──────────────────────────────┐
│ DETERMINE  NORMALITY  OF WELL │────  S125
└──────────────┬───────────────┘
               ↓
┌──────────────────────────────┐
│  ADD  NORMALITY  INFORMATION  │────  S130
│ OF WELL  TO  TEST RESULT DATA │
└──────────────┬───────────────┘
               ↓
        ┌─────────────┐
        │     END     │
        └─────────────┘
```

# FIG. 6

```
                    ┌─────────────┐
                    │   START     │
                    └─────────────┘
                           │
                           ▼
        ┌──────────────────────────────────┐
        │   READ EVALUATION TARGET DATA     │────  S205
        └──────────────────────────────────┘
                           │
                           ▼
        ┌──────────────────────────────────┐
        │   EXCLUDE Cq VALUE OF ABNORMAL    │
        │   WELL FROM EVALUATION TARGET DATA│────  S210
        └──────────────────────────────────┘
                           │
                           ▼
        ┌──────────────────────────────────┐
        │   ACQUIRE EVALUATION TARGET       │
        │   DATA ANALYSIS INFORMATION       │────  S215
        └──────────────────────────────────┘
                           │
                           ▼
        ┌──────────────────────────────────┐
        │      DETERMINE NORMALITY          │────  S220
        └──────────────────────────────────┘
                           │
                           ▼
        ┌──────────────────────────────────┐
        │       READ COMPARISON DATA        │────  S225
        └──────────────────────────────────┘
                           │
                           ▼
        ┌──────────────────────────────────┐
        │   EXCLUDE Cq VALUE OF ABNORMAL    │
        │   WELL FROM COMPARISON DATA       │────  S230
        └──────────────────────────────────┘
                           │
                           ▼
        ┌──────────────────────────────────┐
        │    ACQUIRE COMPARISON DATA        │
        │    ANALYSIS INFORMATION           │────  S235
        └──────────────────────────────────┘
                           │
                           ▼
        ┌──────────────────────────────────┐
        │   ACQUIRE COMPARISON INFORMATION  │────  S240
        └──────────────────────────────────┘
                           │
                           ▼
        ┌──────────────────────────────────┐
        │  OUTPUT ANALYSIS RESULT INFORMATION│────  S245
        └──────────────────────────────────┘
                           │
                           ▼
                    ┌─────────────┐
                    │    END      │
                    └─────────────┘
```

FIG. 7

(a)

(b)

| NUMBER OF COPIES | 1 | 2 | 4 | 8 | 16 | 32 |
|---|---|---|---|---|---|---|
| CV% | 157.7 | 43.7 | 35.5 | 24.4 | 14.3 | 19.2 |
| DETECTION RATIO(%) UD NUMBER | 91.7 2 | 100 0 | 100 0 | 100 0 | 100 0 | 100 0 |

FIG. 8

FIG. 9

# FIG. 10

(a)

|   | 1 | 2 | 3 | 4 | 5 | 6 | 7 | 8 | 9 | 10 | 11 | 12 |
|---|---|---|---|---|---|---|---|---|---|----|----|----|
| A | 34.3 | 34.8 | 35.2 | 34.9 | 34.8 | 34.8 | 34.5 | 34.5 | 34.8 | 35.0 | 34.5 | 34.1 |
| B | 34.4 | 34.8 | 34.3 | 34.7 | 34.7 | 34.4 | 34.8 | 34.8 | 35.1 | 34.1 | 34.4 | 34.8 |
| C | 34.5 | 34.7 | 34.9 | 34.3 | 34.8 | 34.7 | 34.4 | 34.5 | 34.8 | 34.7 | 34.7 | 34.6 |
| D | 34.9 | 34.5 | 34.6 | 34.7 | 34.3 | 34.9 | 34.7 | 34.6 | 34.6 | 34.5 | 35.0 | 35.0 |
| E | 34.4 | 35.0 | 35.0 | 34.8 | 35.0 | 34.5 | 34.6 | 34.7 | 35.0 | 34.6 | 34.9 | 34.6 |
| F | 34.3 | 34.9 | 35.0 | 34.8 | 34.8 | 35.2 | 34.6 | 34.8 | 34.7 | 34.7 | 35.1 | 34.6 |
| G | 34.8 | 35.0 | 34.4 | 34.4 | 34.3 | 35.0 | 34.5 | 34.6 | 34.8 | 34.5 | 35.0 | 35.0 |
| H | 35.0 | 35.1 | 35.0 | 34.5 | 34.9 | 34.8 | 34.5 | 34.5 | 34.7 | 34.7 | 34.7 | 35.0 |

(b)

| NUMBER OF COPIES | 10 |
|---|---|
| Cq Ave | 34.71 |
| $\sigma$ | 0.24 |
| RSD% | 0.69 |
| $\Delta$Cq | 1.13 |
| Max | 35.22 |
| Min | 34.09 |
| UD NUMBER | 0 |
| DETECTION RATIO | 100.0% |

# FIG. 11

(a)

|   | 1 | 2 | 3 | 4 | 5 | 6 | 7 | 8 | 9 | 10 | 11 | 12 |
|---|---|---|---|---|---|---|---|---|---|----|----|----|
| A | 34.7 | 34.7 | 34.8 | 35.0 | 34.9 | 34.9 | 34.9 | 34.6 | 35.0 | 34.8 | 35.2 | 34.5 |
| B | 35.0 | 34.9 | 34.8 | 34.9 | 35.1 | 35.1 | 35.0 | 34.8 | 34.8 | 34.9 | 35.3 | 34.8 |
| C | 34.7 | 34.8 | 35.0 | 35.1 | 35.0 | 35.0 | 35.3 | 35.4 | 35.2 | 34.8 | 35.0 | 35.0 |
| D | 35.4 | 34.9 | 35.0 | 34.8 | 35.0 | 35.3 | 34.9 | 34.9 | 34.7 | 35.0 | 35.1 | 35.0 |
| E | 35.4 | 35.0 | 34.9 | 34.8 | 35.1 | 35.2 | 35.1 | 35.3 | 34.9 | 34.9 | 34.6 | 34.8 |
| F | 34.7 | 35.0 | 34.9 | 34.9 | 35.0 | 34.8 | 35.0 | 35.0 | 34.9 | 34.7 | 35.0 | 34.7 |
| G | 34.8 | 35.2 | 34.8 | 35.0 | 35.0 | 34.8 | 34.8 | 35.1 | 35.0 | 34.7 | 34.8 | 34.6 |
| H | 35.3 | 34.7 | 35.2 | 35.0 | 34.9 | 34.8 | 34.7 | 34.9 | 35.1 | 35.0 | 35.0 | 34.6 |

(b)

| NUMBER OF COPIES | 10 |
|---|---|
| Cq Ave | 34.94 |
| $\sigma$ | 0.19 |
| RSD% | 0.55 |
| $\triangle$Cq | 0.96 |
| Max | 35.43 |
| Min | 34.47 |
| UD NUMBER | 0 |
| DETECTION RATIO | 100.0% |

# FIG. 12

(a)

|  | 1 | 2 | 3 | 4 | 5 | 6 | 7 | 8 | 9 | 10 | 11 | 12 |
|---|---|---|---|---|---|---|---|---|---|---|---|---|
| A | UD | UD | UD | UD | UD | UD | UD | UD | UD | UD | UD | UD |
| B | UD | 35.3 | 34.7 | 33.5 | 32.7 | 31.6 | UD | 35.7 | 34.9 | 33.5 | 32.7 | 31.5 |
| C | UD | 35.9 | 34.8 | 33.8 | 32.5 | 31.6 | 37.8 | 35.3 | 35.8 | 33.8 | 33.0 | 31.5 |
| D | UD | 35.3 | 34.9 | 33.8 | 32.5 | 31.7 | 37.8 | 35.8 | 34.7 | 33.6 | 32.8 | 31.3 |
| E | 37.7 | 35.9 | 34.5 | 33.5 | 32.7 | 31.6 | 38.7 | 35.7 | 34.3 | 33.3 | 32.8 | 31.5 |
| F | 38.7 | 35.6 | 34.6 | 33.4 | 32.6 | 31.6 | 37.7 | 35.4 | 34.6 | 33.7 | 32.8 | 31.4 |
| G | 37.7 | 35.7 | 34.7 | 33.4 | 32.7 | 31.5 | 37.6 | 35.9 | 34.4 | 33.6 | 32.4 | 31.3 |
| H | 37.7 | 36.0 | 34.9 | 33.8 | 32.6 | 31.5 | 37.7 | 36.1 | 34.7 | 33.5 | 32.6 | 31.0 |

(b)

| NUMBER OF COPIES | 1 | 5 | 10 | 20 | 40 | 80 |
|---|---|---|---|---|---|---|
| Cq Ave | 37.91 | 35.69 | 34.74 | 33.6 | 32.68 | 31.48 |
| $\sigma$ | 0.42 | 0.27 | 0.34 | 0.15 | 0.16 | 0.17 |
| RSD% | 1.10 | 0.76 | 0.99 | 0.44 | 0.50 | 0.53 |
| $\triangle$Cq | 1.05 | 0.81 | 1.44 | 0.48 | 0.59 | 0.66 |
| Max | 38.70 | 36.11 | 35.76 | 33.81 | 33.03 | 31.70 |
| Min | 37.65 | 35.30 | 34.32 | 33.33 | 32.43 | 31.04 |
| UD NUMBER | 4 | 0 | 0 | 0 | 0 | 0 |
| DETECTION RATIO | 71.4% | 100.0% | 100.0% | 100.0% | 100.0% | 100.0% |

(c)

$y = -3.3769x + 38.015$
$R^2 = 0.9826$

AMPLIFICATION
EFFICIENCY
97.8%

# FIG. 13

(a)

|   | 1 | 2 | 3 | 4 | 5 | 6 | 7 | 8 | 9 | 10 | 11 | 12 |
|---|---|---|---|---|---|---|---|---|---|----|----|----|
| A | UD | UD | UD | UD | UD | UD | UD | UD | UD | UD | UD | UD |
| B | UD | 36.0 | 34.7 | 33.6 | 32.8 | 31.5 | 37.7 | 35.8 | 34.6 | 33.8 | 32.5 | 31.5 |
| C | UD | 35.4 | 34.3 | 33.6 | 32.6 | 31.6 | 37.7 | 35.5 | 34.7 | 33.2 | 32.8 | 31.5 |
| D | 37.8 | 35.6 | 34.9 | 33.7 | 32.5 | 31.7 | 38.8 | 36.0 | 34.6 | 33.7 | 32.7 | 31.4 |
| E | 38.8 | 35.7 | 34.8 | 33.5 | 31.2 | 31.6 | 36.8 | 35.8 | 34.8 | 33.2 | 32.6 | 31.6 |
| F | UD | 37.0 | 34.9 | 33.6 | 32.6 | 31.5 | 37.7 | 35.3 | 35.0 | 33.8 | 32.6 | 31.5 |
| G | 37.8 | 36.7 | 34.8 | 33.6 | 32.6 | 31.5 | 38.8 | 35.3 | 34.9 | 33.6 | 32.6 | 31.3 |
| H | 36.7 | 35.5 | 35.3 | 33.4 | 32.7 | 31.4 | 37.7 | 35.4 | 34.8 | 33.3 | 32.4 | 31.1 |

(b)

| NUMBER OF COPIES | 1 | 5 | 10 | 20 | 40 | 80 |
|---|---|---|---|---|---|---|
| Cq Ave | 37.84 | 35.79 | 34.78 | 33.54 | 32.51 | 31.49 |
| $\sigma$ | 0.73 | 0.51 | 0.20 | 0.21 | 0.39 | 0.16 |
| RSD% | 1.92 | 1.42 | 0.58 | 0.62 | 1.21 | 0.51 |
| $\Delta$Cq | 2.13 | 1.74 | 0.91 | 0.65 | 1.62 | 0.62 |
| Max | 38.84 | 37.05 | 35.26 | 33.81 | 32.82 | 31.69 |
| Min | 36.71 | 35.31 | 34.35 | 33.16 | 31.20 | 31.08 |
| UD NUMBER | 3 | 0 | 0 | 0 | 0 | 0 |
| DETECTION RATIO | 78.6% | 100.0% | 100.0% | 100.0% | 100.0% | 100.0% |

(c)

$y = -3.402x + 38.022$
$R^2 = 0.9618$

AMPLIFICATION
EFFICIENCY
96.8%

# FIG. 14

## FIG. 15

(a)

(b)

|  | PAST DATA | CURRENT DATA |
|---|---|---|
| Cq Ave | 34. 828 | 34. 886 |
| Cq σ | 0. 435 | 0. 233 |
| △Cq | — | 1. 295 |

FIG. 16

## INTERNATIONAL SEARCH REPORT

| | International application No. |
|---|---|
| | **PCT/JP2021/027866** |

| **A.** | **CLASSIFICATION OF SUBJECT MATTER** |
|---|---|

*C12M 1/00*(2006.01)i; *C12M 1/34*(2006.01)i; *G01N 35/00*(2006.01)i; *C12Q 1/6851*(2018.01)i; *C12Q 1/686*(2018.01)i
FI:    C12M1/00 A; G01N35/00 F; C12M1/34 Z; C12Q1/686 Z; C12Q1/6851 Z

According to International Patent Classification (IPC) or to both national classification and IPC

| **B.** | **FIELDS SEARCHED** |
|---|---|

Minimum documentation searched (classification system followed by classification symbols)

C12M1/00; C12M1/34; G01N35/00; C12Q1/6851; C12Q1/686

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII)

| **C.** | **DOCUMENTS CONSIDERED TO BE RELEVANT** | |
|---|---|---|

| Category* | Citation of document, with indication, where appropriate, of the relevant passages | Relevant to claim No. |
|---|---|---|
| X | JP 2020-054327 A (RICOH CO., LTD.) 09 April 2020 (2020-04-09) claims, in particular, claims 1-3, 7,8, 11, 14-16, paragraphs [0013], [0035], [0051], [0214], [0219], fig. 32, 33 | 1-16 |
| X | JP 2019-092495 A (RICOH CO., LTD.) 20 June 2019 (2019-06-20) claims, in particular, claims 1, 2, 5, 6, 9-12, paragraphs [0013], [0024], [0183], [0185], [0188], fig. 32, 33 | 1-16 |
| X | JP 2019-092506 A (RICOH CO., LTD.) 20 June 2019 (2019-06-20) claims, in particular, claims 1, 2, 5, 6, 10, 13-15, paragraphs [0014], [0037], [0052], [0206], [0209], fig. 21, 22 | 1-16 |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **16 September 2021** | **05 October 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

International application No.

**PCT/JP2021/027866**

| Patent document cited in search report | | | Publication date (day/month/year) | Patent family member(s) | | | Publication date (day/month/year) |
|---|---|---|---|---|---|---|---|
| JP | 2020-054327 | A | 09 April 2020 | US | 2020/0384464 | A1 | |
| | | | | paragraphs [0163], [0165], [0197], [0414], [0499], [0507], [0850]-[0992], fig. 34, 35 | | | |
| | | | | WO | 2019/103128 | A1 | |
| | | | | WO | 2019/093530 | A1 | |
| JP | 2019-092495 | A | 20 June 2019 | US | 2020/0384464 | A1 | |
| | | | | paragraphs [0163], [0165], [0197], [0414], [0499], [0507], [0850]-[0992], fig. 34, 35 | | | |
| | | | | WO | 2019/103128 | A1 | |
| | | | | WO | 2019/093530 | A1 | |
| JP | 2019-092506 | A | 20 June 2019 | US | 2021/0010072 | A1 | |
| | | | | claims, paragraphs [0096], [0099], [150], [0177], [0456], [0464], fig. 32, 33 | | | |
| | | | | US | 2020/0384464 | A1 | |
| | | | | WO | 2019/103128 | A1 | |
| | | | | WO | 2019/093530 | A1 | |

Form PCT/ISA/210 (patent family annex) (January 2015)

## REFERENCES CITED IN THE DESCRIPTION

**Patent documents cited in the description**

- JP 2020130957 A

- JP 2020054327 A

**Non-patent literature cited in the description**

- **A. FOROOTAN et al.** Methods to determine limit of detection and limit of quantification in quantitative real-time PCR (qPCR). *Biomolecular Detection and Quantification,* June 2017, vol. 12, 1-6